# EUROPÄISCHE PATENTSCHRIFT

(11) **EP 4 029 024 B1**
(45) Veröffentlichungstag und Bekanntmachung des Hinweises auf die Patenterteilung: **08.11.2023**
(21) Anmeldenummer: 20767855.8
(22) Anmeldetag: 11.09.2020
(51) Int. Cl.: G16C 20/10, G16C 20/90, G16C 60/00, B01J 19/00, G16C 20/30, G16C 20/70, G06N 3/02

(54) **VERFAHREN UND VORRICHTUNG ZUR UNTERSTÜTZUNG DER HERSTELLUNG EINES FUNKTIONSMATERIALS**
METHOD AND DEVICE FOR SUPPORTING THE PRODUCTION OF A FUNCTIONAL MATERIAL
PROCÉDÉ ET DISPOSITIF D'AIDE À LA FABRICATION D'UNE MATIÈRE FONCTIONNELLE

(30) Priorität: 13.09.2019 EP 19197347
(43) Veröffentlichungstag der Anmeldung: 20.07.2022
(73) Patentinhaber: HTE GmbH The High Throughput Experimentation Company, 69123 Heidelberg (DE)
(72) Erfinder: LEJKOWSKI, Michael Ludwig, 67056 Ludwigshafen (DE); KRAEMER, Michael, 67056 Ludwigshafen (DE); SAUER, Tilman, 69123 Heidelberg (DE); STRASSER, Andreas, 67056 Ludwigshafen (DE); RUPP, Benjamin, 69123 Heidelberg (DE)
(74) Vertreter: BASF IP Association
(86) Internationale Anmeldenummer: PCT/EP2020/075496
(87) Internationale Veröffentlichungsnummer: WO 2021/048372

(56) Entgegenhaltungen:
- WO-A1-2018/035718
- FR-A1- 2 865 819
- US-A1- 2002 086 791
- ANDREAS SUNDERMANN ET AL: "High-Throughput Screening as a Supplemental Tool for the Development of Advanced Emission Control Catalysts: Methodological Approaches and Data Processing", CATALYSTS, Bd. 6, Nr. 2, 1. Februar 2016 (2016-02-01) , Seite 23, XP055668797, CH ISSN: 2073-4344, DOI: 10.3390/catal6020023 in der Anmeldung erwähnt
- LUTZE P ET AL: "Process intensification: A perspective on process synthesis", CHEMICAL ENGINEERING AND PROCESSING, ELSEVIER SEQUOIA, LAUSANNE, CH, Bd. 49, Nr. 6, 1. Juni 2010 (2010-06-01), Seiten 547-558, XP027138842, ISSN: 0255-2701 [gefunden am 2010-05-19] in der Anmeldung erwähnt

## Beschreibung

### Gebiet der Erfindung

Die vorliegende Erfindung betrifft ein Verfahren und eine Vorrichtung, die es Benutzern erlaubt ein Funktionsmaterial wie einen Katalysator entwerfen zu lassen, und insbesondere erlaubt durch die Ausführung zu leiten und alle notwendigen Parameter zu erfassen.

### Hintergrund der Erfindung

Die Aktivität eines Katalysators hängt stark von seiner Herstellungsgeschichte ab. Die Auswahl der Inhaltsstoffe, diverse Parameter verschiedener Verfahrensschritte, die zum fertigen Katalysator führen, und die chemische Zusammensetzung des fertigen Katalysators können einen erheblichen Einfluss auf die Katalysatoraktivität haben.

Es ist daher von hohem Wert, die oben genannten Informationen in einer relationalen Datenbank strukturiert und hierarchisch zu erfassen und zu speichern. Dies bildet die Grundlage für eine spätere Erkennung von Struktur-Wirkungs-Beziehungen.

Aus US 2002/0086791 A1 ist ein maschineller Ansatz bekannt, der einen Ansatz zur Entwicklung von skalierbaren heterogenen Katalysatoren und Festkörpern mit Hochleistungseigenschaften bereitstellt. Dieser maschinelle Ansatz umfasst drei Hauptkomponenten: den Datengenerationszyklus mittels Hochdurchsatzverfahren, den Wissensgenerationszyklus und die Wissensablage oder Datenbank.

Aus WO 2013/175240 ist ein Verfahren zur Herstellung eines Produktes bekannt, das die vom Benutzer erwarteten Eigenschaften treffen oder übertreffen soll, wobei ein Algorithmus zur Optimierung des Verfahrens eingesetzt wird. Bei dem in WO 2013/175240 beschriebenen Verfahren handelt es sich um ein Flusssystem wobei das Analytik-System an das Flusssystem angepasst ist.

Aus A. Sundermann et al., "High-throughput screening as a supplemental tool for the development of advanced emission control catalysts: Methodological approaches and data processing," vol. 6, no. 2, p. 23, Feb. 2016 ist beispielsweise ein Hochdurchsatz-Screening für die Entwicklung fortschrittlicher Katalysatoren zur Emissionskontrolle bekannt.

Aus D. Lutze et al., "Process intensification: A perspective on process synthesis," Chemical engineering and processing, Elsevier Sequoia, vol. 49, no. 6, pp. 547-558, Jun. 2010 ist eine Prozessintensivierung für eine Prozesssynthese bekannt.

Aus WO 2018/035718 A1 ist ein Vorhersagemodell bekannt, das eine genaue Echtzeit-Vorhersage der Produktionsraten chemischer Produkte in chemischen Anlagen ermöglichen soll.

### Zusammenfassung der Erfindung

Der Gegenstand der vorliegenden Erfindung betrifft ein Verfahren und eine Vorrichtung zur Überwachung und Evaluierung einer Herstellung eines Katalysators gemäß den unabhängigen Ansprüchen, weitere Ausführungsformen der Erfindung werden in den anhängigen Ansprüchen verkörpert.

Gemäß eine Ausführungsform der Erfindung wird ein Verfahren bereitgestellt zur Überwachung und Evaluierung einer Herstellung eines Funktionsmaterials, wobei das Funktionsmaterial ein Katalysator ist und das Verfahren aufweist:
Bereitstellen von einer Mehrzahl von vorgegebenen Prozessen an einen Benutzer mit jeweils vorgegebenen Prozessparametern eines Prozesses für die Herstellung eines Funktionsmaterials und dessen Eigenschaften; Erfassen von durch den Benutzer vorgenommenen Schritten bei einem Abarbeiten eines aus der Mehrzahl von Prozessen ausgewählten Prozesses; Vergleichen der durch den Benutzer vorgenommenen Schritte mit den Prozessparametern des ausgewählten Prozesses für die Herstellung eines Funktionsmaterials; Bewerten der durch den Benutzer vorgenommenen Schritte auf der Grundlage einer Datenbasis, die eine Mehrzahl von durch frühere Benutzer abgearbeitete Prozesse mit Prozessparametern für die Herstellung von Funktionsmaterialien und deren analysierten Eigenschaften aufweist; Rückmelden an den Benutzer, in welchem Umfang vorbestimmte Eigenschaften des durch den abgearbeiteten Prozess hergestellten Funktionsmaterials erreicht werden bei Abweichungen der vorgenommenen Schritte bei einer Abarbeitung des ausgewählten Prozesses von den vorgegebenen Prozessparametern des ausgewählten Prozesses.

Auf diese Weise kann einem Benutzer bei der Herstellung von Funktionsmaterialien eine Wissensbasis zur Verfügung gestellt werden, die ihn dabei unterstützt Funktionsmaterialien zu erforschen, deren Parameter zu verwalten und die Ergebnisse zu prognostizieren, um auf diese Weise einen effizienteren Arbeitsablauf bei der systematischen Erforschung und Herstellung von Funktionsmaterialien zu ermöglichen.

Der Begriff Materialien ist nicht einschränkend und umfasst sämtliche Materialien, einschließlich von Eingangs- und Ausgangsstoffen, die in Verbindung mit der Durchführung des Prozesses zur Herstellung eines Katalysators eingesetzt werden. Im Rahmen der vorliegenden Beschreibung werden die Eingangsstoffe auch als Vorläufermaterialien bezeichnet, die wiederum aus den Vorvorläufermaterialien hergestellt werden können, so dass die entsprechenden Prozessparameter, die die unterschiedlichen Materialbeziehungen (der Herstellungshistorie) und die Eigenschaften beschreiben, bei dem Verfahren gespeichert werden können. Grundsätzlich kann man in einem Prozess die chemische Zusammensetzung betrachten, wie auch die Verfahrensschrittparameter (wie z.B. die Calciniertemperatur bei der Calcination), denen chemische Zusammensetzungen unterzogen werden können. Eingangs- und Ausgangsmaterialien können letztlich mit Massenanteilen von (Alias) Komponenten beschrieben werden. Eine Zusammensetzung in Bezug auf die chemischen Elemente lässt sich durch unterschiedliche formelmäßige Zusammenhänge beschreiben. Einzelne Eigenschaften können dann in der Form eines Stoffparameters beschrieben werden. Im Sinne der Erfindung sollen jedoch unter Prozessparametern sowohl Materialparameter, z.B. chemische Zusammensetzungen in Bezug auf die chemischen Elemente bzw. Stoffe oder Materialien, als auch Verfahrensparameter verstanden werden.

Die Datenbasis kann Vergleichsdaten und/oder Referenzdaten aufweisen. Die Datenbasis weist als Vergleichsdaten und/oder Referenzdaten beispielsweise eine Mehrzahl von durch frühere Benutzer abgearbeitete Prozesse mit Prozessparametern für die Herstellung von Funktionsmaterialien und deren analysierten Eigenschaften auf. Vergleichsdaten und/oder Referenzdaten können auch analytisch generierte Daten sein, oder auch Zusammenhänge zwischen Prozessparametern und Eigenschaften, die der Literatur oder früheren Analysen entnommen sind.

Die Parameter, die ein Material charakterisieren, können physikalische und chemische Parameter sein, wobei zu den chemischen Parametern die einzelnen Inhaltsstoffe zählen, die durch die Art und Menge der in den Materialien enthaltenen Elemente angegeben werden und die beispielsweise bei einer Elementaranalyse des Materials bestimmt werden. Weiterhin kann ein Material charakterisiert sein durch einen oder mehrere Parameter, die ausgewählt sind aus der Gruppe Identifizierungsnummer beziehungsweise Registrierungsnummer, Datum der Herstellung, Name des Herstellers, Gebinde Identifizierungsnummer, und/oder Material-ID. Zur Beschreibung von Materialien stehen eine Vielzahl von Parametern zur Verfügung.

Unter früheren Benutzern versteht man in diesem Kontext eine Zuweisung zu einem in der Vergangenheit durchgeführten Prozess, ungeachtet der Person des Benutzers. Insbesondere kann der frühere Benutzer die gleiche Person wie der nunmehr agierende Benutzer sein. Der von dem nunmehr agierenden Benutzer vorgenommene Prozess kann dann auch der Datenbasis zugeführt werden, sodass der Prozess des nunmehr agierenden Benutzers zu einem Prozess eines früheren Benutzers wird.

Das Bewerten kann auch das Registrieren der Eigenschaften und das Registrieren von Abweichungen umfassen, um auf diese Weise dem Benutzer einen Anhaltspunkt zu geben, ob der Prozess voraussichtlich zum gewünschten Erfolg führen wird.

Ein Katalysator kann dabei jedes Material sein, welches eine physikalische, biologische und/oder chemische Funktionalität als Katalysator aufweist. Beispielhaft, aber nicht abschließend können das anorganische Katalysatoren sein, wie zum Beispiel Festkörperkatalysatoren. Anorganische Funktionsmaterialien können mit entsprechenden Leistungseigenschaften ausgestattet sein, zum Beispiel können Festkörperkatalysatoren nach ihrer Selektivität, Aktivität und/oder Standzeit im Prozess unter gegebenen Prozessbedingungen spezifiziert werden.

Unzulässige Abweichungen in Bezug auf Parameter liegen vor, wenn vorgegebene Parameter überschritten werden, wobei der Wert der Überschreitung größer ist als der Wert der Fehlertoleranz. Unzulässige Abweichungen in Bezug auf Materialien liegen vor, wenn eine fehlerhafte Zuordnung erfolgt, was anhand eines Identifizierers erkannt werden kann. Unzulässige Abweichungen können registriert werden. Gleichzeitig kann ein Eingreifen durch den Benutzer vorgenommen werden, um unzulässige Abweichungen zu beseitigen.

Gegenüber dem bekannten Stand der Technik, etwa A. Sundermann et al., "High-throughput screening as a supplemental tool for the development of advanced emission control catalysts: Methodological approaches and data processing," ermöglicht die Erfindung nicht nur ein Prüfen von Funktionsmaterialien, sondern auch die Überwachung der Herstellung. Darüber hinaus ermöglicht die Erfindung gegenüber dem Stand der Technik einen Vergleich der durch den Benutzer vorgenommenen Schritte mit den Prozessparametern des ausgewählten Prozesses für die Herstellung eines Funktionsmaterials oder Funktionswerkstoffes. Dadurch wird ermöglicht die Bewertung der vom Benutzer vorgenommenen Schritte auf der Grundlage einer Datenbank vorzunehmen, die eine Vielzahl von durch frühere Benutzer vorgenommenen Schritten auf der Grundlage einer Datenbasis, die eine Mehrzahl von durch frühere Benutzer abgearbeitete Prozesse mit Prozessparametern für die Herstellung von Funktionsmaterialien und deren analysierten Eigenschaften aufweist. Dadurch kann im Falle einer Abweichung von Prozessparametern des gewählten Verfahrens zur Herstellung eines Funktionsmaterials der daraus resultierende Einfluss auf die Eigenschaften des Funktionsmaterials vorhergesagt und dem Anwender mitgeteilt werden. So wird der Anwender informiert, wenn eine Abweichung von Prozessparametern des gewählten Verfahrens zu einer Veränderung der Eigenschaften des hergestellten Funktionsmaterials führt. Insbesondere, wenn sich die Eigenschaften des hergestellten Funktionsmaterials aufgrund der Abweichung von den Prozessparametern des gewählten Prozesses verschlechtern, wird der Anwender gewarnt und kann in den auszuführenden Prozess eingreifen. Dies ermöglicht einen effizienteren Arbeitsablauf bei der systematischen Recherche und Herstellung von Funktionsmaterialien.

Gemäß einer Ausführungsform der Erfindung kann das Bereitstellen von vorgegebenen Prozessen auch die Möglichkeit umfassen, vorhandene Prozesse abzuändern und/oder Prozesse hinzuzufügen.

Auf diese Weise wird eine höhere Flexibilität des Verfahrens erreicht und eine leichtere Modifikation des Ablaufes ermöglicht.

Gemäß einer Ausführungsform der Erfindung kann das Bereitstellen von vorgegebenen Prozessen auch die Möglichkeit umfassen Arbeitsablaufinstanzen (Workflow Instances) zu erstellen.

Auf diese Weise können ähnliche Proben mit bestimmten Variationen einzelner Parameter eines Workflows dem Verfahren unterzogen werden. Es kann sich dabei um beispielhafte Arbeitsabläufe handeln, bei denen Variationen zugelassen sind. Die Variationen einzelner Parameter kann hilfreich sein, um bestimmte Effekte zu analysieren und kann einen Beitrag liefern zur Optimierung von Funktionsmaterialien.

Gemäß eine Ausführungsform der Erfindung umfasst das Verfahren ferner ein Assistieren für einen Benutzer bei einer Ansatzberechnung, wobei das Assistieren insbesondere ein Berechnen von Verhältnissen und/oder ein Umrechnen von Parametern umfasst, wobei insbesondere zumindest ein Teil der Prozesse skalierbar ist und einem Benutzer die Auswahl zur Dimensionierung dieser Prozesse anbietet. Gemäß einer Ausführungsform kann bei einer Ansatzberechnung eine sogenannte Mehrfach-Verhältnis-Tabelle erstellt werden, und ein Gruppieren von Elementen, (Alias) Komponenten und Materialien vorgesehen sein. Dabei können Gruppen auch in den Verhältnistabellen als Referenz eingesetzt werden.

Auf diese Weise kann der Benutzer eine weiterreichende Unterstützung bei der Herstellung eines Funktionsmaterials erhalten, in dem nicht nur die durchgeführten Prozessschritte erfasst bzw. registriert werden, und der Bewertung unterzogen werden, sondern der Benutzer auch vor und beim Durchführen der Schritte eine Unterstützung erhält, die Fehler vermeidet.

Beim Assistieren kann das Verfahren auch ergänzende Informationen bereitstellen oder auch Suchfunktionen, sowie eine assistierte oder automatisierte Aliasnamenzuweisung. Es können beim Assistieren beispielsweise auch Informationen bereitgestellt werden, die stöchiometrischen Komponenten und Alias Komponenten inkl. Massenanteile beschreiben. Eine Suchfunktion kann auch ein Suchen nach Proben nach ihrer Herstellungsgeschichte (Verfahrensparameter) oder Inhaltsstoffen (Materialparameter, z.B. Fe, Co, Ni, ...) umfassen. Eine Suchfunktion kann auch ermöglichen, die Datenbasis nach ausgewählten Parametern, Prozessparametern, Parametern in der Zusammensetzung zu durchsuchten, beispielsweise eine Suche nach Proben mit Aktivkomponente Platin im Bereich mit 0,05 - 1 Gew.-%, oder eine Suche nach Proben, die mit einem Material eines speziellen Trägeroxids hergestellt wurden.

Gemäß einer Ausführungsform der Erfindung bietet das Verfahren auch die Vergabe von sogenannten Alias-Namen für Platzhalter im Allgemeinen oder auch als Platzhalter für Parameter an.

Auf diese Weise kann eine assistierte Zuordnung erfolgen, die auch das Auffinden eines Materials unter seinem Trivialnamen ermöglicht. Grundsätzlich können Materialien durch mehrere Komponenten beschrieben werden. Eine Komponente kann eine chemische Formel haben, wie z.B. NaCl für Kochsalz. Eine Alias Komponente ist eine Variante der Komponente, die einfach nur einen Namen/Label hat, aber zum Beispiel keine Information über ihre chemische Zusammensetzung haben muss. Komponenten können später in Verhältnissen für die Ansatzberechnung eingesetzt werden. Sind Komponenten durch eine Formel beschrieben, kann sich in den Verhältnissen auch auf die enthaltenen chemischen Elemente bezogen werden.

Gemäß einer Ausführungsform der Erfindung umfasst das Verfahren ferner ein Überwachen eines Benutzers bei dem Abarbeiten der vorgenommenen Schritte eines aus der Mehrzahl von Prozessen ausgewählten Prozesses.

Auf diese Weise kann frühzeitig ermittelt werden, ob der Benutzer eine Fehlbedienung vornimmt oder nicht prozessgemäß arbeitet, bzw. sich außerhalb von vorbestimmten Regeln verhält.

Gemäß einer Ausführungsform der Erfindung umfasst das Überwachen ein Vergleichen wenigstens eines durch den Benutzer beabsichtigten Schrittes mit einem entsprechenden Prozessparameter eines ausgewählten Prozesses für die Herstellung eines Katalysators, sowie eine Warnung bei einer unzulässigen Abweichung.

Auf diese Weise kann bei einer Abweichung eine unmittelbare Rückmeldung an den Benutzer gegeben werden oder auch an andere Personen oder Überwachungseinrichtungen, die dann ggf. in den abzuarbeitenden Prozess eingreifen können.

Gemäß einer Ausführungsform der Erfindung umfasst das Überwachen ein Lesen eines Identifizierers eines Vorratsbehälters eines von einem Benutzer vorgesehenen Materials und ein Vergleich mit einem für den entsprechenden vorgegebenen Prozess vorgesehenen Material, sowie eine Warnung bei einer unzulässigen Abweichung.

Auf diese Weise kann auch die Material- bzw. Stoffauswahl des Benutzers automatisiert überwacht werden, insbesondere, um menschliche Fehlleistungen, wie ein "sich verlesen" oder "sich vergucken" zu kompensieren. Dies kann auch eine robotergestützte Verfahrensweise unterstützen.

Gemäß einer Ausführungsform der Erfindung kann zur Identifizierung den einzelnen Materialien eine Identifizierungs-Nummer beziehungsweise eine Registrierungsnummer zugeordnet werden. Es ist anzumerken, dass jeder einzelnen Probe eine eigene Identifizierungs-Nummer zugeordnet wird, so dass beispielsweise ein pulverförmiges Trägeroxid eine andere ID-Nummer erhält als diejenige Probe, die als Teilmenge zur Weiterverarbeitung aus der Gesamtprobe entnommen wird. Somit kann jedes Material und sämtliche mit dem Material verbundenen Proben in einer auffindbaren Weise in der Datenbasis hinterlegt werden.

Gemäß einer Ausführungsform der Erfindung umfasst ein Prozessparameter bzw. ein durch den Benutzer vorgenommener Schritt wenigstens einen Materialparameter und einen Verarbeitungsparameter und/oder einen Herkunftsparameter eines Materials.

Auf diese Weise können die Prozessschritte definiert werden, durch Materialauswahl oder Verfahrensschrittauswahl, wie Temperatur oder Behandlungsdauer, ausgewählt werden.

Gemäß einer Ausführungsform der Erfindung umfasst das Bewerten der durch den Benutzer vorgenommenen Schritte eine Anwendung einer künstlichen Intelligenz, die auf der Grundlage der in der Datenbasis abgelegten Mehrzahl von durch frühere Benutzer abgearbeiteten Prozesse für die Herstellung von Katalysatoren und deren gemessenen Eigenschaften eine Korrelation von wenigstens einem Prozessparameter eines Prozesses für die Herstellung eines Katalysators und wenigstens einer Eigenschaft des Katalysators herstellt.

Auf diese Weise kann ein zusätzlicher Informationsgewinn erzielt werden, der über eine reine Kombinatorik hinausgeht. Jeder weitere der Datenbasis zugeführte Prozess kann ermöglichen weitere Korrelationen zu identifizieren, sodass in einem genaueren Maße vorhergesagt werden kann, welche Auswirkungen bestimmte Parameter haben und welche Auswirkungen bestimmte Abweichungen von diesen Parametern haben können. Dadurch kann die Anzahl der Versuche reduziert werden, die erforderlich sind, ein bestimmtes Funktionsmaterial mit den gewünschten Eigenschaften herzustellen.

Gemäß einer Ausführungsform der Erfindung wird auf der Grundlage der Korrelation und der durch den Benutzer vorgenommenen Schritte bei einer Abarbeitung des ausgewählten Prozesses eine Prognose für wenigstens eine Eigenschaft des herzustellenden Katalysators erstellt.

Auf diese Weise kann eine konkrete Prognose erstellt werden, welche Eigenschaften ein Funktionsmaterial voraussichtlich haben wird, wenn es nach einem auszuführenden Prozess hergestellt wird.

Gemäß einer Ausführungsform der Erfindung umfasst das Bewerten der durch den Benutzer vorgenommenen Schritte eine Zwischenbewertung, auf der Grundlage eines Zwischenerzeugnisses, auf der Grundlage einer Datenbasis, die eine Mehrzahl von durch frühere Benutzer abgearbeiteten Prozesse für die Herstellung von Vorläufermaterialien und deren gemessenen Eigenschaften aufweist.

Auf diese Weise kann bereits bei Zwischenstufen und Zwischenstadien evaluiert werden, ob diese gewisse gewünschte Eigenschaften aufweisen und ob absehbar ist, ob auch das Endmaterial die gewünschten Eigenschaften ausweisen wird. Dadurch kann andererseits auch frühzeitig ein Prozess abgebrochen werden, wenn am Vorläufermaterial bereits abzusehen ist, dass das Endmaterial die gewünschten Eigenschaften nicht erhalten wird.

Als Vorläufermaterial wird ein Material bezeichnet, welches als Grundlage zur Herstellung eines Funktionsmaterials verwendet wird. Vorläufermaterialien können in verschiedenen Generationen eines mehrstufigen Prozesses vorkommen. Vorläufermaterialien können bereits Eigenschaften aufweisen, die auch das endgültige Funktionsmaterial aufweist. So kann zum Beispiel ein pulverförmiger Katalysator, der mittels Extrudieren in einen Formkörperkatalysator umgewandelt wird, ein Vorläufermaterial sein.

Gemäß einer Ausführungsform der Erfindung wird ein Verfahren bereitgestellt zum Aufbau einer Datenbasis zur Anwendung eines Verfahrens zur Überwachung und Evaluierung einer Herstellung eines Funktionsmaterials, wobei das Verfahren zum Aufbau einer Datenbasis umfasst: Erfassen von durch Benutzer vorgenommenen Schritten bei einer Abarbeitung eines Prozesses zur Herstellung eines Funktionsmaterials; Analysieren von Eigenschaften des durch den Prozess zur Herstellung eines Funktionsmaterials hergestellten Funktionsmaterials; Zuweisen der Eigenschaften des durch den Prozess zur Herstellung eines Funktionsmaterials hergestellten Funktionsmaterials zu den entsprechenden Schritten bei einer Abarbeitung des Prozesses zur Herstellung eines Funktionsmaterials; und Ablegen des Ergebnisses des Zuweisens in der Datenbasis.

Auf diese Weise kann eine Datenbasis aufgebaut werden, die als Wissensgrundlage dient bei der Herstellung von Funktionsmaterialien. Daten von vorherigen Prozessen werden gesammelt, sodass eine Korrelation zwischen den Prozessparametern wie Materialien und Verfahrensparametern und den Eigenschaften des fertigen Materials hergestellt werden kann. Diese Korrelation kann dann auch auf den laufenden Prozess bei der Herstellung eines Funktionsmaterials angewendet werden.

Gemäß einer Ausführungsform der Erfindung umfasst das Verfahren zum Aufbau einer Datenbasis ein Anwenden einer künstlichen Intelligenz, die auf der Grundlage einer Mehrzahl von Erfassungen von durch Benutzer vorgenommenen Schritten bei einer Abarbeitung eines Prozesses zur Herstellung eines Funktionsmaterials und dessen analysierten Eigenschaften eine Korrelation von wenigstens einem Prozessparameter eines Prozesses für die Herstellung eines Funktionsmaterials und wenigstens einer Eigenschaft des analysierten Funktionsmaterials herstellt.

Auf diese Weise kann beispielsweise ein neuronales Netzwerk aufgebaut und verwendet werden, um den "Wissensgehalt" der Datenbasis zu erhöhen. Dies führt zu einer wesentlich höheren Effizienz bei der Herstellung eines Funktionsmaterials.

Gemäß einer Ausführungsform der Erfindung führt das erfindungsgemäße Verfahren das Bewerten der durch den Benutzer vorgenommenen Schritte unter einer Anwendung einer künstlichen Intelligenz durch.

Im Allgemeinen wird bei dem Begriff der künstlichen Intelligenz zwischen einer starken künstlichen Intelligenz und einer schwachen künstlichen Intelligenz unterschieden. Mit einer starken künstlichen Intelligenz wird versucht, den Menschen als Person zu simulieren. Der Begriff schwache künstlichen Intelligenz bezieht sich auf Mittel von selbstständigen Lernprozessen, die Optimierung durch Anwendung von vorgegebenen Algorithmen oder neuronalen Netzwerken, insbesondere auch die Mustererkennung.

Gemäß einer Ausführungsform der Erfindung wird eine Vorrichtung zur Überwachung und Evaluierung einer Herstellung eines Funktionsmaterials bereit gestellt, wobei die Vorrichtung umfasst: eine Benutzerschnittstelle; eine Verarbeitungseinheit; einen Katalog mit einer Mehrzahl von vorgegebenen Prozessen mit jeweils vorgegebenen Prozessparametern eines Prozesses für die Herstellung eines Funktionsmaterials; und eine Datenbasis mit einer Mehrzahl von durch frühere Benutzer abgearbeiteten Prozessen für die Herstellung von Funktionsmaterialien und deren analysierten Eigenschaften; wobei die Benutzerschnittstelle dazu eingerichtet ist dem Benutzer den Katalog zur Auswahl zur Verfügung zu stellen und dem Benutzer eine Auswahl eines Prozesses für die Herstellung eines Funktionsmaterials zu ermöglichen; wobei die Benutzerschnittstelle dazu eingerichtet ist durch den Benutzer vorgenommene Schritte bei einem Abarbeiten eines aus der Mehrzahl von Prozessen ausgewählten Prozesses zu erfassen; wobei die Verarbeitungseinheit dazu eingerichtet ist die durch den Benutzer vorgenommenen Schritte mit den Prozessparametern eines ausgewählten Prozesses für die Herstellung eines Funktionsmaterials zu vergleichen und die durch den Benutzer vorgenommenen Schritte auf der Grundlage der Datenbasis hinsichtlich voraussichtlich erreichter Eigenschaften des Funktionsmaterials zu bewerten; wobei die Benutzerschnittstelle dazu eingerichtet ist an den Benutzer zurückzumelden, in welchem Umfang vorbestimmte Eigenschaften des durch den abgearbeiteten Prozess hergestellten Funktionsmaterials erreicht werden bei Abweichungen der vorgenommenen Schritte bei einer Abarbeitung des ausgewählten Prozesses von den vorgegebenen Prozessparametern des ausgewählten Prozesses.

Auf diese Weise kann eine Vorrichtung bereitgestellt werden, die den Herstellungsprozess eines Funktionsmaterials unterstützt. Der Benutzer kann durch die Vorrichtung unterstützt werden, in dem die Vorrichtung dem Benutzer gewisse Auswahlen zur Verfügung stellt und andere Auswahlen einschränkt, die gemäß vorgegebenen Regeln nicht zielführend sind. Insbesondere kann auch vermieden werden, dass vermeidbare Fehler bei der Berechnung, der Dosierung und der Auswahl von Materialien und Verfahrensparametern passieren.

Gemäß einer Ausführungsform der Erfindung umfasst die Vorrichtung eine Überwachungsvorrichtung, die dazu eingerichtet ist wenigstens einen durch den Benutzer beabsichtigten Schritt mit einem entsprechenden Prozessparameter eines ausgewählten Prozesses für die Herstellung eines Funktionsmaterials zu vergleichen und eine Warnung bei einer unzulässigen Abweichung an den Benutzer auszugeben.

Auf diese Weise kann bei einer Abweichung eine unmittelbare Rückmeldung an den Benutzer gegeben werden oder auch an andere Personen oder Überwachungseinrichtungen, die dann ggf. in den abzuarbeitenden Prozess eingreifen können.

Gemäß einer Ausführungsform der Erfindung umfasst die Vorrichtung eine Lesevorrichtung für einen Identifizierer eines Vorratsbehälters eines von einem Benutzer vorgesehenen Materials, wobei die Vorrichtung ausgeführt ist ein Vergleich mit einem für den entsprechenden vorgegebenen Prozess vorgesehenen Materials vorzunehmen, sowie eine Warnung bei einer unzulässigen Abweichung abzugeben.

Auf diese Weise kann auch die Material- bzw. Stoffauswahl des Benutzers automatisiert überwacht werden, insbesondere um menschliche Fehlleistungen, wie ein "sich verlesen" oder "sich vergucken" zu kompensieren. Dies kann auch eine robotergestützte Verfahrensweise unterstützen.

Gemäß einer Ausführungsform der Erfindung weist die Überwachungsvorrichtung eine Leseeinheit auf, die dazu eingerichtet ist einen Identifizierer eines Vorratsbehälters eines von einem Benutzer vorgesehenen Materials zu lesen, wobei die Überwachungsvorrichtung dazu eingerichtet ist den Inhalt des Identifizierers mit einem für den entsprechenden vorgegebenen Prozess vorgesehenen Material zu vergleichen, und eine Warnung bei einer unzulässigen Abweichung an den Benutzer abzugeben.

Auf diese Weise kann automatisch erfasst werden, ob das richtige Material ausgewählt wurde und bei einer Fehlauswahl eine Warnmeldung ausgegeben werden. Anstelle eines Identifizierers kann auch eine direkte Analyse des zuzuführenden Materials erfolgen und in Abhängigkeit vom Analyseergebnis ggf. eine Warnung abgegeben werden.

Gemäß einer Ausführungsform der Erfindung umfasst die Verarbeitungseinheit eine künstlichen Intelligenz, die dazu eingerichtet ist auf der Grundlage der in der Datenbasis abgelegten Mehrzahl von durch frühere Benutzer abgearbeiteten Prozesse für die Herstellung von Funktionsmaterialien und dessen gemessenen Eigenschaften eine Korrelation von wenigstens einem Prozessparameter eines Prozesses für die Herstellung eines Funktionsmaterials und wenigstens einer Eigenschaft des Funktionsmaterials herzustellen.

Auf diese Weise kann beispielsweise ein neuronales Netzwerk aufgebaut und verwendet werden, um den "Wissensgehalt" der Datenbasis zu erhöhen. Dies führt zu einer wesentlich höheren Effizienz bei der Herstellung eines Funktionsmaterials.

Gemäß einer Ausführungsform der Erfindung wird auf der Grundlage der Korrelation und der durch den Benutzer vorgenommenen Schritte bei einer Abarbeitung des ausgewählten Prozesses eine Prognose für wenigstens eine Eigenschaft des herzustellenden Funktionsmaterials erstellt.

Auf diese Weise kann die Vorrichtung den Herstellungsprozess signifikant beschleunigen, insbesondere, da ein Herstellungsprozess abgekürzt werden kann, wenn absehbar ist, dass dieser aufgrund der Prognose nicht zum gewünschten Erfolg führt.

Diese und andere Merkmale werden anhand der folgenden Figurenbeschreibung erläutert.

### Kurze Beschreibung der Figuren

- Figur 1: zeigt einen schematischen Ablauf eines Verfahrens zur Überwachung und Evaluierung einer Herstellung eines Funktionsmaterials gemäß einer Ausführungsform der Erfindung;
- Figur 2: zeigt eine detailliertere Struktur der Prozesse bzw. eines Prozesskatalogs des in Figur 1 gezeigten Verfahrens gemäß einer Ausführungsform der Erfindung;
- Figur 3: zeigt eine Gegenüberstellung eines Prozessbaumdiagramms aus Figur 2 mit einem Prozess mit Prozessparametern gemäß einer Ausführungsform der Erfindung;
- Figur 4: zeigt einen gegenüber dem in der Figur 1 gezeigten Verfahrensablauf weiter detaillierten Verfahrensablauf zur Überwachung und Evaluierung einer Herstellung eines Funktionsmaterials gemäß einer Ausführungsform der Erfindung;
- Figur 5: zeigt einen schematischen Ablauf eines Verfahrens zum Aufbau einer Datenbasis für ein Verfahren zur Überwachung und Evaluierung einer Herstellung eines Funktionsmaterials gemäß einer Ausführungsform der Erfindung; und
- Figur 6: zeigt die Struktur eine Vorrichtung zur Überwachung und Evaluierung einer Herstellung eines Funktionsmaterials gemäß einer Ausführungsform der Erfindung.

Weitere Merkmale und Vorteile der erfindungsgemäßen Verfahren und der Vorrichtung ergeben sich aus den Figuren und aus der zugehörigen Figurenbeschreibung. Es versteht sich, dass die vorstehend genannten und die nachstehend noch zu erläuternden Merkmale nicht nur in der jeweils angegebenen Kombination, sondern auch in anderen Kombinationen oder in Alleinstellung anwendbar sind ohne den Rahmen der vorliegenden Erfindung zu verlassen. Ausführungsbeispiele der Erfindung sind in den Figuren dargestellt und werden nachfolgend im Detail beschrieben.

### Detaillierte Beschreibung beispielhafter Ausführungsformen

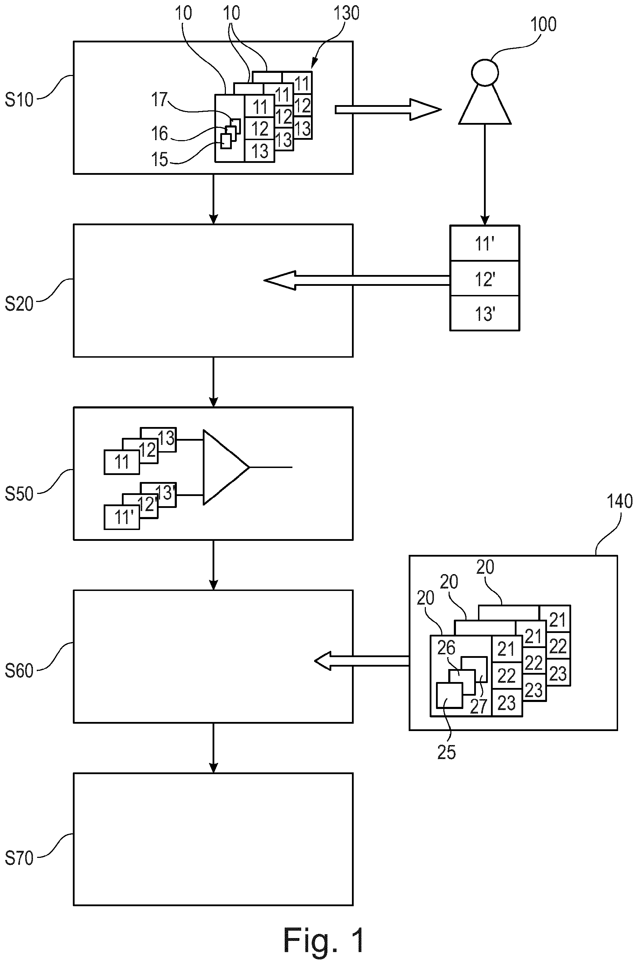

Figur 1 zeigt einen schematischen Ablauf des Verfahrens zur Überwachung und Evaluierung einer Herstellung eines Funktionsmaterials gemäß einer Ausführungsform der Erfindung.

In Schritt S10 erfolgt eine Bereitstellung eines Kataloges 130 mit mehreren Prozessen 10 zur Herstellung eines Funktionsmaterials, wie zum Beispiel eines Katalysators. Die Prozesse sind jeweils definiert durch Prozessparameter 11, 12, 13. Hinter den Prozessen 10 sind auch die Eigenschaften 15, 16, 17 des durch den jeweiligen Prozess 10 hergestellten Funktionsmaterials hinterlegt. Der Benutzer 100 kann aus den zur Verfügung gestellten Prozessen einen Prozess 10 auswählen, der zu dem gewünschten Funktionsmaterial führt. Der ausgewählte Prozess kann auch als Grundlage dienen den ausgewählten Prozess 10 zu modifizieren. Die vom Benutzer vorgenommenen Schritte 11', 12', 13', die den Prozessparametern des tatsächlich abgearbeiteten Prozesses entsprechen, werden dann in Schritt S20 erfasst. Die tatsächlich vorgenommenen Schritte 11', 12', 13' können von den Prozessparametern 11, 12, 13 des ausgewählten Prozesses 10 abweichen, was dann möglicherweise zu einer anderen Eigenschaft des Funktionsmaterials führt. Diese Abweichung kann gewollt sein, etwa durch eine gezielte Modifizierung des Prozesses 10, oder ungewollt, zum Beispiel durch ein Versehen im Abarbeiten oder durch Messungenauigkeiten bei Materialien oder Verfahrensparametern. Die tatsächlich durchgeführten Schritte 11', 12', 13' werden dann mit den vorgesehenen Schritten bzw. Prozessparametern 11, 12, 13 im Schritt S50 abgeglichen bzw. verglichen. Das Vergleichsergebnis wird dann als Grundlage verwendet, um dieses im Schritt S60 mit einer Datenbasis 140 abzugleichen, in der Prozesse 20 abgelegt sind, d.h. Prozessparameter 21, 22, 23, die bei der Herstellung von Funktionsmaterialien zu bestimmten Eigenschaften 25, 26, 27 geführt haben. Die Datenbasis 140 kann dabei eine Vielzahl von Prozessen 20 beinhalten, die von früheren Benutzern durchgeführt wurden. Auf der Grundlage dieser Prozesse 20 kann abgeleitet werden, ob der angestrebte Prozess 10 zum gewünschten Erfolg führt oder nicht. Die Datenbasis 140 kann dabei jedoch nicht nur aus realen früheren Prozessen 20 bestehen, sondern auch Korrelationen enthalten, die aus Prozessen abgeleitet wurden, Zusammenhänge zwischen Prozessparametern 21, 22, 23 und Eigenschaften 25, 26, 27, die auf einer analytischen Ermittlung beruhen, oder auch auf der Anwendung einer künstlichen Intelligenz. Im Schritt S70 erfolgt dann eine Rückmeldung an den Benutzer, ob das gewünschte Ergebnis erreicht wird, in welchem Umfang es erreicht wird, und/oder welche Maßnahmen ergriffen werden müssen, um das gewünschte Ergebnis zu erreichen.

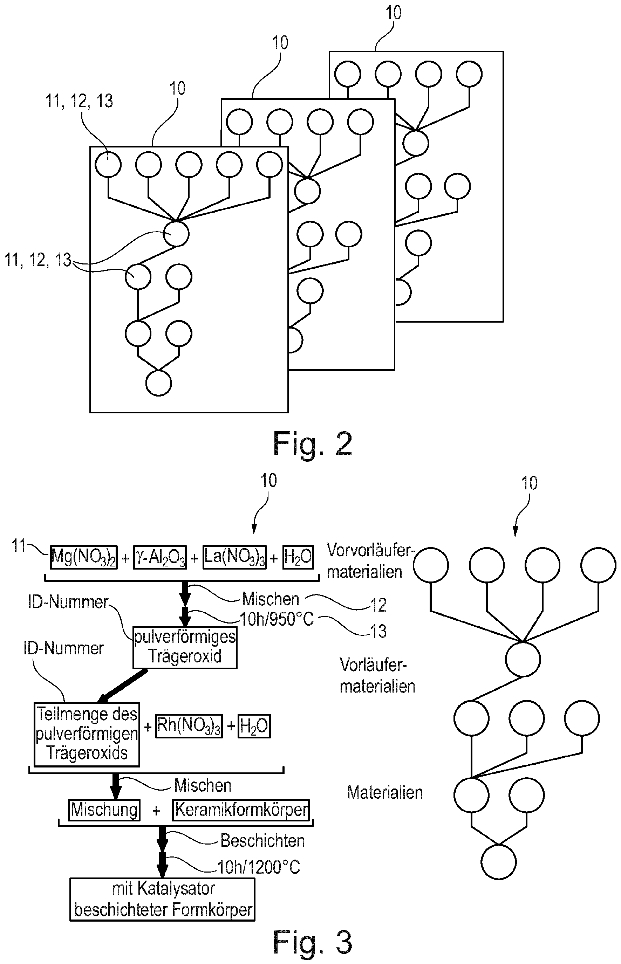

Figur 2 zeigt eine detailliertere Struktur der Prozesse bzw. eines Prozesskatalogs des in Figur 1 gezeigten Verfahrens gemäß einer Ausführungsform der Erfindung. Dabei zeigt Figur 2, dass der Prozess eine verzweigte Baumstruktur aufweisen kann. In einer frühen Ebene (oberer Reihe) können unterschiedliche Ausgangspunkte bzw. Prozessparameter 11, 12, 13 gewählt werden, etwa unterschiedliche Materialien. Diese können dem Benutzer im Schritt S10 zu Auswahl gestellt werden. In den weiteren Ebenen kann es zu Verzweigungen kommen, die je nach ausgewählten Prozessparametern in einer späteren Ebene (weiter unten) ausgewählt werden. Das können Materialauswahlen oder auch Verarbeitungsauswahlen sein, wie etwa die Auswahl einer Behandlungstemperatur oder einer Behandlungszeit. Diese können dem Benutzer ebenfalls zur Auswahl gestellt werden. Dabei kann die Auswahl gemäß vorgegebener Regeln beschränkt werden auf bestimmte Parameter. So können beispielsweise Parameterauswahlen 11, 12, 13, die aufgrund des "Wissens" der Datenbasis 140 zu keinem vielversprechenden Ergebnis 15, 16, 17 führen, dem Benutzer durch das Verfahren vorenthalten werden. Im weiteren Verlauf können auch weitere Materialien dem Prozess zugeführt werden, je nachdem, was im ausgewählten Prozess 10 hinterlegt ist.

Figur 3 zeigt eine Gegenüberstellung eines Prozessbaumdiagramms aus Figur 2 mit einem Prozess 10 mit Prozessparametern 11, 12, 13 gemäß einer Ausführungsform der Erfindung. Figur 3 zeigt links einen beispielhaften Prozess und rechts den Prozess in einer Baumstruktur abgebildet. Die vertikale Achse zeigt den Ablauf bei einer Herstellung von Materialien, wobei einzelne Prozessparameter, z.B. zugeführte Materialien und Prozessschritte, durch ein kreisförmiges Symbol gekennzeichnet sind. Im oberen Bereich sind Ausgangsmaterialien gekennzeichnet, die zur Herstellung eines Funktionsmaterials führen, das anhand des Kreises im unteren Bereich angedeutet wird. Die dazwischenliegenden Kreise können Vorgängermaterialien repräsentieren, wenn diese als Eingangsparameter für einen weiteren Prozessschritt verwendet werden. Zur Illustration von einem Prozess 10 ist in Fig. 3 der schematische Ablauf eines mehrstufigen Verfahren zur Herstellung eines beschichteten Rhodiumkatalysators auf einem keramischen Formkörper gezeigt. In dem Beispiel wird die Verwandtschaftsbeziehungen der Materialien und deren Verbindungen mit Vorläufermaterialien und Vorvorläufermaterialien verdeutlicht. In dem Beispiel geht es um die Herstellung von Oxidationskatalysatoren, bei dem ein Aluminiumoxid als Aluminium-Quelle eingesetzt wird, das zunächst zu einem pulverförmigen Mischoxid umgesetzt wird. Im Anschluss daran wird ein Teil des pulverförmigen Mischoxids mit Aktivkomponente (im vorliegenden Fall Rhodiumnitrat) mit Wasser gemischt und die Mischung zur Beschichtung eines Keramikformköpers verwendet. Nach der Beschichtung wird der Formköper einer thermischen Behandlung unterworfen.

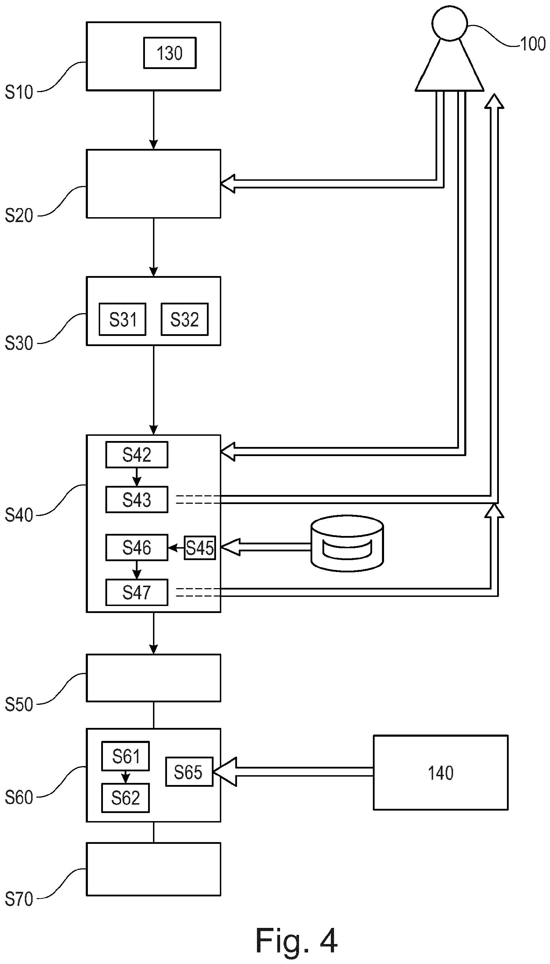

Figur 4 zeigt einen gegenüber dem in der Figur 1 gezeigten Verfahrensablauf weiter detaillierten Verfahrensablauf zur Überwachung und Evaluierung einer Herstellung eines Funktionsmaterials gemäß einer Ausführungsform der Erfindung. Das Verfahren kann ferner ein Assistieren S30 für einen Benutzers 100 bei einer Ansatzberechnung umfassen, wobei das Assistieren insbesondere ein Berechnen S31 von Verhältnissen und/oder ein Umrechnen S32 von Parametern umfasst. Dabei kann insbesondere zumindest ein Teil der Prozesse 10 skalierbar sein und einem Benutzer 100 die Auswahl zur Dimensionierung dieser Prozesse 10 angeboten werden. Dem Benutzer können zahlreiche Tools bereitgestellt werden, die ihm die Auswahl und die Bemessung der Prozessparameter 11, 12, 13 erleichtern. Bei einer Ansatzberechnung können sogenannte Mehrfach-Verhältnis-Tabellen (multiple ratio tables) und das Gruppieren von Elementen, (Alias) Komponenten und Materialien vorgesehen sein. Gruppen können auch in den Verhältnistabellen (ratio tables) als Referenz eingesetzt werden. So kann zum Beispiel die Gruppe "Edelmetalle" aus Rhodium, Platin und Palladium bestehen. Dazu kann dann in einer Mehrfach-VerhältnisTabelle angegeben werden, dass diese Elemente in ein molares Verhältnis von 1:2:3 gesetzt werden. Die Gruppe "Edelmetalle" kann man dann in einer anderen Verhältnistabelle mit Wasser und einem Trägermaterial in eine gewichtsbasierte Beziehung setzen, zum Beispiel: "Edelmetalle":Wasser:Träger = 1:50:100. Darauf basierend können dann benötigten Mengen der Eingangsstoffe für eine bestimmte Ansatzgröße berechnet werden.

Materialien können durch Import aus dem Inventar des Systems oder direktes Neuanlegen in sogenannten Synthesis Request definiert werden oder direkt in ihrer chemischen Zusammensetzung beschrieben werden. Ein Material kann mehrere Komponenten enthalten, die durch chemische Formeln beschrieben werden, zum Beispiel dadurch, dass die molare Masse des Materials automatisch berechnet wird und/oder die Massenanteile pro Komponente automatisch anhand der Reinheit beziehungsweise dem Vorliegen von Verunreinigungen, insbesondere der Art und Menge an Verunreinigungen, berechnet werden. Diese Informationen sind für spätere Ansatzberechnungen wichtig. Materialien können auch direkt mit Komponenten und Massenanteilen beschrieben werden. Weiterhin ist eine Beschreibung mit Alias Komponenten möglich, die keine chemische Zusammensetzung besitzen, wie z.B. "Verunreinigung". Es können weitere Eigenschaften für Materialien eingegeben werden, die spätere Ansatzberechnungen beeinflussen, wie z.B. Konzentration, Dichte, Loss on Ignition (LOI), und/oder Lösungsmittelaufnahme.

Ein Rezepteditor kann vorgesehen sein und ist in erster Linie für den Wissenschaftler gedacht, der die Herstellung von Proben plant. Er bietet zwei große Funktionalitäten: eine Ansatzberechnung von Zutaten für eine Präparation und eine Beschreibung des Herstellungsverfahrens über vorkonfigurierte Prozessschritte mit Parametern. In der Ansatzberechnung kann man zwischen verschiedenen Berechnungsmodi wählen. Diese Modi bieten weitere Auswahloptionen an, die die Ansatzberechnung beeinflussen. In einem zweiten Schritt können die Eingangsmaterialien ausgewählt werden. Hierbei sind alle Materialen aus einem Materialien-Reiter/Menü verfügbar sowie Materialien, die im aktuellen Synthesis Request geplant bzw. hergestellt worden sind. Um die Ansatzberechnung zu steuern, kann man weiterhin folgende Mengenangaben machen: Gesamtmenge aller Eingangsmaterialien, Gesamtmenge des Zielprodukts (ggf. unter Berücksichtigung einer erwarteten Ausbeute), und/oder Mengen einzelner Eingangsmaterialien. Anschließend kann man weitere Randbedingungen für die Ansatzberechnungen vorgeben, wie z.B. Verhältnisse von bestimmen Materialien, Komponenten, Alias Komponenten oder chemischen Elementen.

Bei einem Berechnungsmodus "Chemische Lösung" kann man ein Eingangsmaterial als Lösungsmittel auswählen. Bei einem Berechnungsmodus "Imprägnierung" kann man ein Eingangsmaterial als Träger und ein weiteres als Lösungsmittel auswählen. Die Lösungsmittelaufnahme des Trägers beeinflusst die Berechnung der benötigten Lösungsmittelmenge. Der LOI (der sogenannte loss of ignition) des Trägers wird für die vorgegebene Beladung des Trägers berücksichtigt. Bei dem LOI handelt es sich um den Glühverlust, der beispielsweise in Verbindung mit dem Ausheizen eines Trägers ermittelt wird. Basierend auf diesen Randbedingungen wird die benötige Menge der Eingangsmaterialien berechnet. Falls keine Berechnung wegen fehlender oder widersprüchlicher Eingaben möglich ist, wird der Nutzer im Reiter/Menü "Mitteilungen" informiert. Das Ergebnis der Berechnung kann angezeigt werden. Es handelt sich dabei um die nötigen Einwaagen der Eingangsmaterialien, die den eingegebenen Randbedingungen genügen.

Die Prozessschritte können in einer separaten Administrationsansicht von Nutzern mit bestimmten Rechten konfiguriert werden. Zusätzlich zu den Parametern können auch noch Hilfsberechnungen angeboten werden. Beispielsweise kann ein Verfahrensschritt "Monolith Coating" die Parameter "start weight", "wet weight" und "dry weight" haben. Den Beutzer interessiert am Ende die Gewichtszunahme nach dem Coating. In der Administrationsansicht kann man Formeln hinterlegen, die auf die Parameter des Verfahrensschrittes zugreifen können, beispielsweise "dry gain after coating [g]" = "dry weight" abzüglich "start weight". Diese Hilfsberechnungen können als read-only Werte während der Ausführung angezeigt werden, sobald die für die Rechnung nötigen Parameter einen Wert vom Nutzer bekommen haben. Dies hilft dem Nutzer zu entscheiden, ob er die Parameter noch weiter verändern muss oder nicht. Prozessschritte können einen Namen haben und einen bestimmten Vorgang wie z.B. Filtration oder Calcinierung beschreiben. Ein Prozessschritt kann mehrere Parameter haben, die durch einen Namen, einen Wert mit einem bestimmten Datentyp (Zeichenkette, Textbaustein, ganze Zahl, numerischer Wert, Equipment oder Material) und für Zahlenwerte durch eine Einheit gekennzeichnet sind. Ein Equipment kann selber wiederrum eigene Parameter haben, wie z.B. eine Rührerdrehzahl für einen Drehmixer. Für die Verwaltung von Equipment gibt es eine separate Administrationsansicht. Bestimmte Parameter legen fest, dass durch einen Prozessschritt ein Nebenprodukt entsteht. Beispiele dafür sind Prozessschritte wie Sieben oder Zentrifugieren. Eine Ansatzberechnung und eine beliebige Abfolge von Prozessschritten können in einem sogenannten Workflow miteinander verknüpft werden. Ein Workflow beschreibt also welche Eingangsmaterialien in welchen Verhältnissen benötigt werden und welche Prozessschritte durchlaufen werden, um die gewünschte Ergebnisprobe zu erhalten. Workflows können folgende Elemente: beinhalten: Name; Ansatzberechnung mit Rechenmodus, Auswahl von Eingangsmaterialien und Angaben über die Verhältnisse der Eingangsmaterialien bzw. ihrer Komponenten oder chemischen Elemente. In einem graphischen Editor können mehrere Prozessschritte per Drag & Drop in einen bestehenden Workflow gezogen werden. Diese sollen später in der Durchführung in einer chronologischen Reihenfolge durchgeführt werden. Für jeden Prozessschritt kann man Sollwerte für die Parameter eingeben. Dabei kann man bei numerischen und ganzzahligen Werten, die Einheit umstellen, z.B. von g auf kg. Bereits eingegebene Werte werden dann umgerechnet. Weiterhin kann man Kommentare für einzelne Parameter oder für den gesamten Prozessschritt eingeben.

Eine neue Probe kann ein neues Material ergeben oder an ein bestehendes Material gespeichert werden. Der Name eines neuen Materials kann über eine Formel mit Variablen und Textelementen vergeben werden. Weiterhin können Berechnungseigenschaften und die Zusammensetzung der Ergebnisprobe eingegeben werden. In bestimmen Fällen kann diese Zusammensetzung automatisch berechnet werden. Werden in einem Workflow die insgesamt benötigten Mengen an Eingangsmaterialien ausgerechnet, kann man pro Material Portionen aufteilen. Dies kann nötig sein, wenn man bestimmte Mengen zeitabhängig zugeben möchte. Man kann Gewichtsverhältnisse für die Portionen eingeben. Die erzeugten Portionen kann man in den Prozessschritten des Workflows als Parameter vom Typ Material auswählen, d.h. man kann angeben wann welche Portion benutzt werden soll. Mit einer globalen Berechnung kann man eine Ansatzberechnung ohne zugehörige Prozessschritte durchführen und ohne, dass eine Ergebnisprobe entsteht. Als Berechnungsmodus steht hier ein physikalisches Mischen nach Gewichtsverhältnissen zur Verfügung. Die Berechnung geteilter Portionen ist möglich. Für geteilte Portionen kann auch eine Zielmenge angegeben werden. Diese Eingabe skaliert die gewählte Portion, ohne die anderen Portionen zu beeinflussen. Die berechneten Mengen pro Material kann man in späteren Workflows einsetzen, wobei die berechnete Menge automatisch mit übernommen wird.

Darüber hinaus zeigt Figur 4, dass das Verfahren ein Überwachen S40 der durch den Benutzer 100 vorgenommenen Schritte 11', 12' 13' umfasst, bei dem wenigstens einer durch den Benutzer beabsichtigten Schritte 11', 12', 13' mit einem entsprechenden Prozessparameter 11, 12, 13 eines ausgewählten Prozesses 10 verglichen wird S42. Damit kann die Herstellung eines Funktionsmaterial, zum Beispiel eines Katalysators überwacht werden und eine Warnung S43 bei einer unzulässigen Abweichung abgegeben werden. Dies kann beispielsweise dann erfolgen, wenn der Benutzer einen falschen Schritt ausgeführt hat, und/oder das Ergebnis eines Schrittes nicht zu erwünschten Ergebnis führen würde. Zu diesem Zweck kann auch vorgesehen sein, dass ein Identifizierer eines Vorratsbehälters gelesen wird 45, der ein von einem Benutzer vorgesehenes Material beinhaltet, und dieses mit einem für den entsprechenden vorgegebenen Prozess vorgesehenen Material verglichen wird S46. Gegebenenfalls kann bei einer unerwarteten Abweichung eine Warnung abgegeben werden S47.

Figur 4 zeigt weiterhin, dass das Bewerten S60 der durch den Benutzer 100 vorgenommenen Schritte eine Anwendung einer künstlichen Intelligenz S65 umfasst, die auf der Grundlage der in der Datenbasis abgelegten Mehrzahl von durch frühere Benutzer abgearbeiteten Prozessen 20 für die Herstellung von Funktionsmaterialien und deren gemessenen Eigenschaften 25, 26, 27 eine Korrelation von wenigstens einem Prozessparameter 11, 12, 13 eines Prozesses 10 für die Herstellung eines Funktionsmaterials und wenigstens einer Eigenschaft des Funktionsmaterials 25, 26, 27 herstellt.

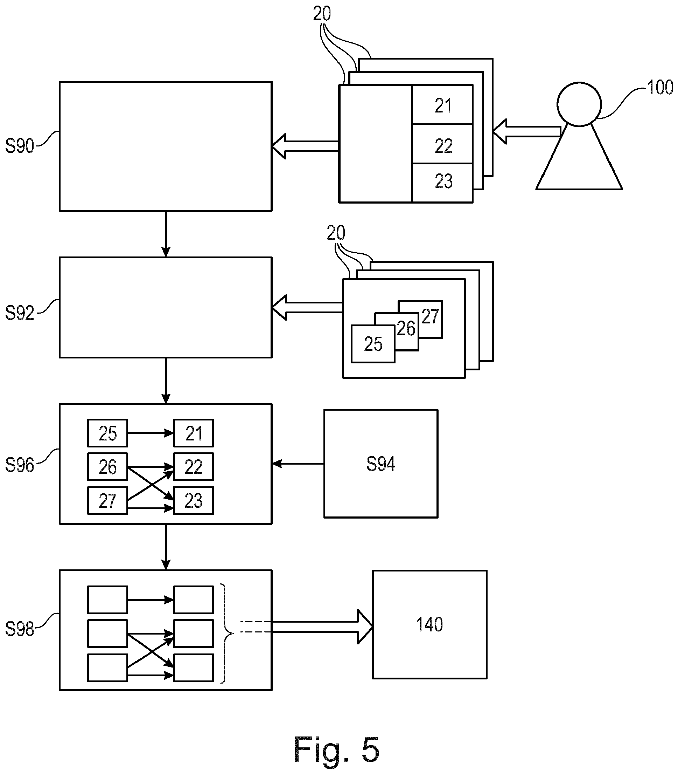

Figur 5 zeigt einen schematischen Ablauf eines Verfahrens zum Aufbau einer Datenbasis für ein Verfahren zur Überwachung und Evaluierung einer Herstellung eines Funktionsmaterials gemäß einer Ausführungsform der Erfindung. Dabei werden durch Benutzer vorgenommene Schritte 21, 22, 23 bei einer Abarbeitung eines Prozesses 20 zur Herstellung eines Funktionsmaterials erfasst S90. Die Eigenschaften 25, 26, 27 des durch den Prozess 20 zur Herstellung eines Funktionsmaterials hergestellten Funktionsmaterialien werden analysiert S92 und die Eigenschaften 25, 26, 27 des durch den Prozess 20 zur Herstellung eines Funktionsmaterials hergestellten Funktionsmaterials den entsprechenden Schritten 21, 22, 23 bei einer Abarbeitung des Prozesses 20 zur Herstellung eines Funktionsmaterials zugewiesen S96. Die Zuweisung kann dabei linear erfolgen, wie in Figur 5 beispielhaft zwischen der Eigenschaft 25 und dem Parameter 21 gezeigt ist, oder auch korreliert, wie zwischen den Ergebnissen 26 und 27 einerseits und den Parametern 22 und 23 andererseits. Die Ergebnisse der Zuweisung werden dann in der Datenbasis 140 abgelegt S98. Die Zuweisung kann auf der Grundlage von früheren Prozessen 20 erfolgen, jedoch auch auf der Grundlage von analytisch herbeigeführten Zusammenhängen zwischen den Prozessparametern 21, 22, 23 und den Eigenschaften 25, 26, 27 des entsprechenden Funktionsmaterials. Die Zuweisung kann auch auf der Grundlage einer angewendeten künstlichen Intelligenz erfolgen, bei der beispielsweise mittels einem neuronalen Netzwerk Zusammenhänge erkannt werden und dadurch eine Zuweisung erfolgt.

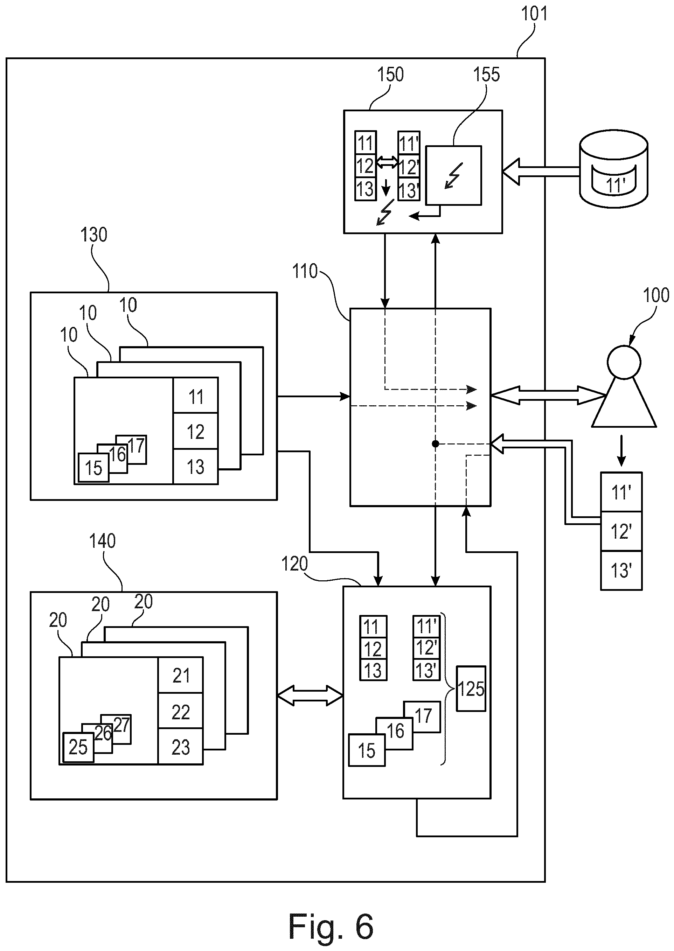

Figur 6 zeigt die Struktur einer Vorrichtung zur Überwachung und Evaluierung einer Herstellung eines Funktionsmaterials gemäß einer Ausführungsform der Erfindung. Die Vorrichtung weist eine Benutzerschnittstelle 110 auf, über die der Benutzer mit der Vorrichtung kommunizieren kann und über die die Vorrichtung Informationen an den Benutzer abgeben kann. Die Benutzerschnittstelle 110 stellt dem Benutzer 100 den Katalog 130 mit den Prozessen 10 bzw. die Menüs zur Auswahl von Prozessparametern zur Verfügung für die Herstellung eines Funktionsmaterials. Die Benutzerschnittstelle 110 kann auch die durch den Benutzer vorgenommene Schritte 11', 12', 13' bei einem Abarbeiten eines aus der Mehrzahl von Prozessen ausgewählten Prozesses 10 erfassen. Darüber hinaus ist eine Verarbeitungseinheit 120 vorgesehen. Die Verarbeitungseinheit 120 kann die durch den Benutzer vorgenommenen Schritte 11', 12', 13' mit den Prozessparametern 11, 12, 13 eines ausgewählten Prozesses 10 für die Herstellung eines Funktionsmaterials vergleichen und die durch den Benutzer vorgenommenen Schritte 11', 12', 13' auf der Grundlage der Datenbasis 140 hinsichtlich voraussichtlich erreichter Eigenschaften des Funktionsmaterials bewerten. Der Vorgang kann iterativ ausgestaltet sein, sodass dem Benutzer immer wieder eine Unterstützung angeboten werden kann. Die Verarbeitungseinheit 120 erhält vom Katalog 130 die entsprechenden Informationen der Auswahl des Benutzers 100 und von der Benutzerschnittstelle 110 die Auswahl des Benutzers und die Handlungen des Benutzers, insbesondere die vom Benutzer ausgeführten Schritte. Die Verarbeitungseinheit 120 ist mit der Datenbasis 140 verbunden, in der eine Mehrzahl von durch frühere Benutzer abgearbeitete Prozesse 20 für die Herstellung von Funktionsmaterialien und deren analysierten Eigenschaften 25, 26, 27 abgelegt ist. Die Verarbeitungseinheit 120 vergleicht die durch den Benutzer vorgenommenen Schritte 11', 12', 13' mit den Prozessparametern 11, 12, 13 eines ausgewählten Prozesses 10 für die Herstellung eines Funktionsmaterials und bewertet die durch den Benutzer vorgenommenen Schritte 11', 12', 13' auf der Grundlage der Datenbasis 140 hinsichtlich voraussichtlich erreichter Eigenschaften des Funktionsmaterials. Die Benutzerschnittstelle 110 meldet an Benutzer zurück, in welchem Umfang vorbestimmte Eigenschaften 15, 16, 17 des durch den abgearbeiteten Prozess 10 hergestellten Funktionsmaterials erreicht werden bei Abweichungen der vorgenommenen Schritte 11', 12', 13' bei einer Abarbeitung des ausgewählten Prozesses von den vorgegebenen Prozessparametern 11, 12, 13 des ausgewählten Prozesses 10. Darüber hinaus weist die Vorrichtung eine Überwachungsvorrichtung 150 auf, die einen durch den Benutzer 100 beabsichtigten Schritt 11`, 12', 13' mit einem entsprechenden Prozessparameter 11, 12, 13 eines ausgewählten Prozesses 10 für die Herstellung eines Funktionsmaterials vergleicht und eine Warnung bei einer unzulässigen Abweichung an den Benutzer ausgibt. Zu diesem Zweck kann die Überwachungseinheit 150 eine Leseeinheit 155 aufweisen, die einen Identifizerer 11' auf einem Materialbehälter lesen kann, der in diesem Fall einen Benutzerschritt darstellt, um so einen materialbezogenen Prozessparameter zu erfassen und diesen dem Vergleich mit dem entsprechenden Prozessparameter 11 des Prozesses 10 zuführen. Die Verarbeitungseinheit 120 kann eine künstlichen Intelligenz 125 umfassen, die dazu eingerichtet ist auf der Grundlage der in der Datenbasis 140 abgelegten Mehrzahl von durch frühere Benutzer abgearbeiteten Prozesse 20 eine Korrelation von wenigstens einem Prozessparameter 21, 22, 23 eines Prozesses 20 für die Herstellung eines Funktionsmaterials und wenigstens einer Eigenschaft 25, 26, 27 des Funktionsmaterials herzustellen. Die künstliche Intelligenz 125 kann dabei auch auf die erfassten Parameter 11', 12`,13` angewendet werden, um dadurch die Eigenschaften 15, 16, 17 zu prognostizieren. Dabei kann auf der Grundlage einer Korrelation und der durch den Benutzer vorgenommenen Schritte 11', 12', 13' bei einer Abarbeitung des ausgewählten Prozesses 10 eine Prognose für wenigstens eine Eigenschaft 15, 16, 17 des herzustellenden Funktionsmaterials erstellt werden.

Das Verfahren der Erfindung zur Überwachung und Evaluierung einer Herstellung eines Funktionsmaterials kann durch eine Client-Server Architektur mit einer zentralen Datenbank für die Datenablage und -abfrage realisiert werden. Der Client kann eine Windows Applikation sein, die auf dem Rechner des Benutzers gestartet wird und sich mit dem Server verbindet. Nutzer können sog. "Synthesis Requests" in einer zweistufigen Ordnungshierarchie von sog. Projekten und Studien anlegen. Diese Synthesis Requests enthalten mehrere Bestandteile, zum Beispiel: Allgemeine Informationen über den Synthesis Request, wie zum Beispiel Projektleiter, Kontaktpersonen, gewünschtes Fertigstellungsdatum; Materialien, die für die Präparation benötigt werden; und/oder einen Rezepteditor. Insbesondere ist zur Planung ein Reiter/Menü vorhanden, in dem der Benutzer die Verfahrenseigenschaften und/oder Anweisungen in einer Karte anklicken kann.

An jedem Prozessschritt kann eine Zwischenprobe genommen werden, die analog zu einer Ergebnisprobe beschrieben wird. Dies kann von Interesse sein, falls man für die Zwischenprobe eine Analyse durchführen möchte. Für die Berechnung der Zielmenge kann man pro Eingangsmaterial festlegen, ob das gesamte Material oder einzelne/mehrere Komponenten zur Zielmenge beitragen. Weiterhin kann ein Eingangsmaterial auch gar nicht zur Zielmenge beitragen.

Da man in der Hochdurchsatzforschung häufig mehrere Proben auf eine ähnliche Art unter Variation bestimmter Faktoren herstellen möchte, kann man mehrere Instanzen eines Workflows anlegen. Jede Workflow Instanz kann eine separate Ergebnisprobe erzeugen. Weiterhin kann man Faktoren einführen, die es erlauben, Mengen, Materialien oder Verhältnisfaktoren zu variieren. Auch die Beschreibung der Ergebnisprobe kann man über Faktoren abbilden. Parameter von Prozessschritten können auch über einen Faktor variiert werden. Die Level für die Faktoren kann man in der Übersichtstabelle der Workflow Instanzen direkt eingeben.

Das Verknüpfen der Zielmenge einer Workflow Instanz erlaubt, dass genauso viel von der Ergebnisprobe erzeugt wird, wie in nachfolgenden Workflow Instanzen insgesamt als Eingangsmaterial verbraucht wird.

Die Präparation kann für eine oder mehrere Instanzen eines Workflows gleichzeitig bearbeitet werden. Es gibt drei Hauptaufgaben während der Ausführung: Eingabe der Auswaagen der Eingangsmaterialien, Eingabe der Ist-Parameter der Prozessschritte, und Eingabe der Gewichte und Lagerorte der Ergebnisproben. Jede Workflow Instanz hat einen Status und erst gestartet und danach beendet werden. Der Ablauf weist eine hohe Flexibilität auf, was die Änderung der Verfahrensabläufe betrifft. Hierbei sind Unterbrechungen oder ein Abbruch der Durchführung des Ablaufs möglich, wenn beispielsweise Eingangsmaterialien nicht im Bestand vorhanden sind oder eine Änderung der Priorisierung vorgenommen wird, die dazu führt, dass der Ablauf unterbrochen wird. Beim Starten einer Workflow Instanz wird der aktuelle Stand aus dem Rezepteditor für diese Workflow Instanz kopiert. Nachträgliche Änderungen am Rezept haben keinen Effekt auf eine gestartete Workflow Instanz. Eine gestartete Workflow Instanz kann aber editiert werden, falls der Bearbeiter bemerkt, dass die ursprüngliche Planung nicht funktioniert. Beispielsweise können Eingangsmaterialien nicht in der gewünschten Menge vorliegen, es muss ein Eingangsmaterial von einem anderen Hersteller verwendet werden oder es müssen Prozessschritte hinzugefügt oder entfernt werden, die vorher nicht abzusehen waren. Es öffnet sich in einer Benutzeroberfläche ein modales Fenster, das in seiner Funktion an den Rezepteditor erinnert. Änderungen können mit einem Klick auf "Fertig stellen" übernommen werden. Die Auswaage der Eingangsmaterialien kann in einer Tabelle für mehrere gestartete und markierte Workflow Instanzen eingegeben werden. Dabei werden Barcodes beim Auswiegen und die Ist-Gewichte erfasst. Stimmt der Barcode nicht mit dem geplanten überein, gibt es eine Warnung. Die Differenz zwischen Soll- und Ist-Gewicht wird angezeigt. Das System ist verbunden mit einem System zur Chemikalienlagerhaltung, bei dem Eingangsregistrierung, Vorratshaltung und Entnahmedaten erfasst werden, auf das das Verfahren im Ablauf zurückgreift. Bei der Durchführung des Verfahrens kommt es zur Entnahme von Materialien, die von dem Verfahren in der Datenbasis erfasst werden. Somit kann der Benutzer über die Erfordernisse von Nachbestellungen informiert werden. Wird ein Material für eine bestimmte Workflow Instanz nicht benötigt, ist die entsprechende Zelle in der Tabelle ausgegraut. Die Eingabe der Ist-Werte für die Parameter der Prozessschritte kann auch in einer Tabellenansicht erfolgen, die man nach verschiedenen Spalten per Drag&Drop gruppieren kann. Standardmäßig wird nach Prozessschritt und dem Index der Workflow Instanz gruppiert. Auf dem weitern Reiter/Menü können Informationen über das Gewicht der Probe durch Eingabe von Tara und Brutto Gewicht oder direkte Eingabe der Ist-Menge und über den Lagerort eingegeben werden. Jeder Lagerort kann zentral im System registriert sein und diese können auf einer speziellen Administrationsseite von Nutzern mit entsprechenden Zugriffsrechten verwaltet werden. Außerdem kann eine automatische Inventur der Einsatzstoffe vorgenommen werden, sodass beispielsweise, wenn von einer Probe A, die mit 550 g vorliegt und 100 g abgewogen werden für
eine Präparation, die Menge von Probe A automatisch auf 450 g aktualisiert wird.

Jeder Synthesis Request hat einen Status. Dabei gibt es folgende Möglichkeiten: Synthese anfordern, dort kann der Synthese Request in diesem Status von einem Labormitarbeiter begutachtet werden; Synthese starten, nachdem die Synthese angefordert wurde, erst dann ist es möglich in der Ausführung einzelne Workflow Instanzen zu starten; und Synthese abschließen, nachdem alle Workflow Instanzen beendet sind, er gilt damit als vollständig bearbeitet.

Für die Labormitarbeiter, die die Synthesis Requests abarbeiten, kann eine Übersichtsansicht im System vorgesehen sein, in der man alle Synthesis Requests nach Status und weiteren Kriterien filtern und sortieren kann. Die Übersicht kann weiterhin mit einer Freitextsuche durchsucht werden. Jeder einzelne Synthesis Request kann durch einen Doppelklick geöffnet werden. Ungeöffnete Synthesis Requests können in fetter Schrift dargestellt werden. Beim Proben erstellen kann folgende Informationen abgespeichert werden: Die Relation der Ergebnisprobe zu den verwendeten Eingangsmaterialien; Reihenfolge und Namen der Prozessschritte; Soll- und Istwerte der Parameter der Prozessschritte inkl. Namen und Einheit; Auswaagen der Eingangsmaterialien inklusive der Einheit; und eine Menge inklusive der Einheit und Lagerort der Ergebnisproben.

Proben können nach vielen Merkmalen in der Datenbank gesucht werden. Diese können mit logischen Verknüpfungen in Beziehung gesetzt werden. Basierend auf solch einer Probensuche kann man sich die o.g. gespeicherten Informationen anzeigen lassen.

### Bezugszeichenliste:

- 10: Prozess, Katalogprozess
- 11: Prozessparameter, Katalogprozessparameter
- 12: Prozessparameter, Katalogprozessparameter
- 13: Prozessparameter, Katalogprozessparameter
- 11': Prozessschritt eines Benutzers
- 12': Prozessschritt eines Benutzers
- 13': Prozessschritt eines Benutzers
- 15: Eigenschaft eines (Katalog-)Prozesserzeugnisses
- 16: Eigenschaft eines (Katalog-)Prozesserzeugnisses
- 17: Eigenschaft eines (Katalog-)Prozesserzeugnisses
- 20: Prozess eines früheren Benutzers (Lernprozess)
- 21: Prozessparameter eines Lernprozesses
- 22: Prozessparameter eines Lernprozesses
- 23: Prozessparameter eines Lernprozesses
- 25: Eigenschaft eines Produktes eines Lernprozesses
- 26: Eigenschaft eines Produktes eines Lernprozesses
- 27: Eigenschaft eines Produktes eines Lernprozesses
- 100: Benutzer
- 101: Vorrichtung zum Überwachen/Evaluierung einer Herstellung eines Katalysators
- 110: Benutzerschnittstelle
- 120: Verarbeitungseinheit
- 125: Künstliche Intelligenz-Einheit
- 130: Prozesskatalog
- 140: Datenbasis
- 150: Überwachungseinheit
- 155: Leseeinheit
- S10: Bereitstellen von Prozessen/eines Prozesskataloges
- S20: Erfassen von Benutzerschritten
- S30: Assistieren eines Benutzers
- S31: Berechnen von Verhältnissen
- S32: Umrechnen von Parametern
- S40: Überwachen eines Benutzers
- S42: Vergleichen von durch den Benutzer beabsichtigten Schritt mit Prozessparameter
- S43: Warnung an Benutzer
- S45: Lesen eines Identifizierers eines Vorratsbehälters
- S46: Vergleich Identifizierer mit Prozessparameter/Material
- S47: Warnung an Benutzer
- S50: Vergleichen von Benutzerschritten mit Prozessparametern
- S60: Bewerten der durch den Benutzer vorgenommenen Schritte
- S61: Zwischenbewertung
- S62: Zwischenbewertung
- S65: Anwendung künstlicher Intelligenz auf Bewertungsprozess
- S70: Rückmeldung an den Benutzer
- S90: Erfassung von Prozessschritten/Parametern frühere Benutzer
- S92: Analysieren von Eigenschaften früherer Prozessergebnisse/Katalysatoren
- S94: Anwendung künstlicher Intelligenz auf Aufbau der Datenbasis
- S96: Zuweisung von Eigenschaften eines Prozesserzeugnisses zu Prozessparametern
- S98: Ablegen der Zuweisungsergebnisse in Datenbasis

## Patentansprüche

1. Computer-implementiertes Verfahren zur Überwachung und Evaluierung einer Herstellung eines Funktionsmaterials, wobei das Funktionsmaterial ein Katalysator ist, und wobei das Verfahren aufweist:
Bereitstellen (S10) von einer Mehrzahl von vorgegebenen Prozessen (10) an einen Benutzer (100) mit jeweils vorgegebenen Prozessparametern (11, 12, 13) eines Prozesses (10) für die Herstellung eines Katalysators und dessen Eigenschaften (15, 16, 17),
Erfassen (S20) von durch den Benutzer vorgenommenen Schritten (11', 12', 13') bei einem Abarbeiten eines aus der Mehrzahl von Prozessen ausgewählten Prozesses (10),
Vergleichen (S50) der durch den Benutzer vorgenommenen Schritte (11', 12', 13') mit den Prozessparametern (11, 12, 13) des ausgewählten Prozesses (10) für die Herstellung eines Katalysators,
Bewerten (S60) der durch den Benutzer vorgenommenen Schritte (11', 12', 13') auf der Grundlage einer Datenbasis, die eine Mehrzahl von durch frühere Benutzer abgearbeitete Prozesse (20) mit Prozessparametern (21, 22, 23) für die Herstellung von Katalysatoren und deren analysierten Eigenschaften (25, 26, 27) aufweist,
Rückmelden (S70) an den Benutzer, in welchem Umfang vorbestimmte Eigenschaften (15, 16, 17) des durch den abgearbeiteten Prozess (10) hergestellten Katalysators erreicht werden bei Abweichungen der vorgenommenen Schritte (11', 12', 13') bei einer Abarbeitung des ausgewählten Prozesses von den vorgegebenen Prozessparametern (11, 12, 13) des ausgewählten Prozesses (10).

2. Verfahren gemäß Anspruch 1, wobei das Verfahren ferner ein Assistieren (S30) für einen Benutzer (100) bei einer Ansatzberechnung umfasst, wobei das Assistieren insbesondere ein Berechnen (S31) von Verhältnissen und/oder ein Umrechnen (S32) von Parametern umfasst, wobei insbesondere zumindest ein Teil der Prozesse (10) skalierbar ist und einem Benutzer (100) die Auswahl zur Dimensionierung dieser Prozesse (10) anbietet.

3. Verfahren gemäß einem der Ansprüche 1 und 2, wobei das Verfahren ferner ein Überwachen (S40) eines Benutzers (100) bei dem Abarbeiten der vorgenommenen Schritte eines aus der Mehrzahl von Prozessen ausgewählten Prozesses (10) umfasst.

4. Verfahren gemäß Anspruch 3, wobei das Überwachen (S40) ein Vergleichen (S42) wenigstens eines durch den Benutzer beabsichtigten Schrittes (11', 12', 13') mit einem entsprechenden Prozessparameter (11, 12, 13) eines ausgewählten Prozesses (10) für die Herstellung eines Katalysators, sowie eine Warnung (S43) bei einer unzulässigen Abweichung umfasst.

5. Verfahren gemäß einem der Ansprüche 3 und 4, wobei das Überwachen (S40) ein Lesen (S45) eines Identifizierers eines Vorratsbehälters eines von einem Benutzer vorgesehenen Materials und ein Vergleich (S46) mit einem für den entsprechenden vorgegebenen Prozess vorgesehenen Materials, sowie eine Warnung (S47) bei einer unzulässigen Abweichung umfasst.

6. Verfahren gemäß einem der vorherigen Ansprüche, wobei ein Prozessparameter bzw. ein durch den Benutzer vorgenommener Schritt (11, 11', 12, 12', 13, 13') wenigstens einen Materialparameter und einen Verarbeitungsparameter umfasst, und/oder einen Herkunftsparameter eines Materials umfasst.

7. Verfahren gemäß einem der vorherigen Ansprüche, wobei das Bewerten (S60) der durch den Benutzer (100) vorgenommenen Schritte eine Anwendung (S65) einer künstlichen Intelligenz umfasst, die auf der Grundlage der in der Datenbasis abgelegten Mehrzahl von durch frühere Benutzer abgearbeiteten Prozesse (20) für die Herstellung von Katalysatoren und deren gemessenen Eigenschaften (25, 26, 27) eine Korrelation von wenigstens einem Prozessparameter (11, 12, 13) eines Prozesses (10) für die Herstellung eines Katalysators und wenigstens einer Eigenschaft des Katalysators (25, 26, 27) herstellt.

8. Verfahren gemäß Anspruch 7, wobei auf der Grundlage der Korrelation und der durch den Benutzer vorgenommenen Schritte (11', 12', 13') bei einer Abarbeitung des ausgewählten Prozesses (10) eine Prognose für wenigstens eine Eigenschaft (15, 16, 17) des herzustellenden Funktionsmaterials erstellt wird.

9. Verfahren gemäß einem der vorherigen Ansprüche, wobei das Bewerten (S60) der durch den Benutzer (100) vorgenommenen Schritte eine Zwischenbewertung (S61, S62) auf der Grundlage eines Zwischenerzeugnisses umfasst auf der Grundlage einer Datenbasis, die eine Mehrzahl von durch frühere Benutzer abgearbeiteten Prozessen für die Herstellung von Vorläufermaterialien und deren gemessenen Eigenschaften aufweist.

10. Computer-implementiertes Verfahren zum Aufbau einer Datenbasis zur Anwendung eines Verfahrens gemäß einem der Ansprüche 1 bis 10, wobei das Verfahren umfasst:
Erfassen (S90) von durch Benutzer vorgenommenen Schritten (21, 22, 23) bei einer Abarbeitung eines Prozesses (20) zur Herstellung eines Katalysators,
Analysieren (S92) von Eigenschaften (25, 26, 27) des durch den Prozess (20) zur Herstellung eines Katalysators hergestellten Katalysators,
Zuweisen (S96) der Eigenschaften (25, 26, 27) des durch den Prozess (20) zur Herstellung eines Katalysators hergestellten Katalysators zu den entsprechenden Schritten (21, 22, 23) bei einer Abarbeitung des Prozesses (20) zur Herstellung eines Katalysators,
Ablegen (S98) des Ergebnisses des Zuweisens in der Datenbasis.

11. Verfahren gemäß Anspruch 10, wobei das Verfahren zum Aufbau einer Datenbasis ein Anwenden (S94) einer künstlichen Intelligenz umfasst, die auf der Grundlage einer Mehrzahl von Erfassungen (S90) von durch Benutzer (100) vorgenommenen Schritten (21, 22, 23) bei einer Abarbeitung eines Prozesses (20) zur Herstellung eines Katalysators und deren analysierten Eigenschaften (25, 26, 27) eine Korrelation von wenigstens einem Prozessparameter (21, 22, 23) eines Prozesses (20) für die Herstellung eines Katalysators und wenigstens einer Eigenschaft (25, 26, 27) des analysierten Katalysators herstellt.

12. Vorrichtung zur Überwachung und Evaluierung einer Herstellung eines Funktionsmaterials, wobei das Funktionsmaterial ein Katalysator ist, und wobei die Vorrichtung umfasst:
eine Benutzerschnittstelle (110),
eine Verarbeitungseinheit (120),
einen Katalog (130) mit einer Mehrzahl von vorgegebenen Prozessen (10) mit jeweils vorgegebenen Prozessparametern (11, 12, 13) eines Prozesses für die Herstellung eines Katalysators, und
eine Datenbasis (140) mit einer Mehrzahl von durch frühere Benutzer abgearbeiteten Prozessen (20) für die Herstellung von Katalysatoren und deren analysierten Eigenschaften (25, 26, 27),
wobei die Benutzerschnittstelle (110) dazu eingerichtet ist dem Benutzer (100) den Katalog (130) zur Auswahl zur Verfügung zu stellen und dem Benutzer eine Auswahl eines Prozesses (10) für die Herstellung eines Katalysators zu ermöglichen,
wobei die Benutzerschnittstelle (110) dazu eingerichtet ist durch den Benutzer vorgenommene Schritte (11', 12', 13') bei einem Abarbeiten eines aus der Mehrzahl von Prozessen ausgewählten Prozesses (10) zu erfassen,
wobei die Verarbeitungseinheit (120) dazu eingerichtet ist die durch den Benutzer vorgenommenen Schritte (11', 12', 13') mit den Prozessparametern (11, 12, 13) eines ausgewählten Prozesses (10) für die Herstellung eines Katalysators zu vergleichen und die durch den Benutzer vorgenommenen Schritte (11', 12', 13') auf der Grundlage der Datenbasis (140) hinsichtlich voraussichtlich erreichter Eigenschaften des Katalysators zu bewerten,
wobei die Benutzerschnittstelle (110) dazu eingerichtet ist an den Benutzer zurückzumelden, in welchem Umfang vorbestimmte Eigenschaften (15, 16, 17) des durch den abgearbeiteten Prozess (10) hergestellten Katalysators erreicht werden bei Abweichungen der vorgenommenen Schritte (11', 12', 13') bei einer Abarbeitung des ausgewählten Prozesses von den vorgegebenen Prozessparametern (11, 12, 13) des ausgewählten Prozesses (10).

13. Vorrichtung gemäß Anspruch 12, wobei die Vorrichtung eine Überwachungsvorrichtung (150) aufweist, die dazu eingerichtet ist wenigstens einen durch den Benutzer (100) beabsichtigten Schritt (11', 12', 13') mit einem entsprechenden Prozessparameter (11, 12, 13) eines ausgewählten Prozesses (10) für die Herstellung eines Katalysators zu vergleichen und eine Warnung bei einer unzulässigen Abweichung an den Benutzer auszugeben.

14. Vorrichtung gemäß einem der Ansprüche 12 bis 13, wobei die Verarbeitungseinheit (120) eine künstlichen Intelligenz (125) umfasst, die dazu eingerichtet ist auf der Grundlage der in der Datenbasis (140) abgelegten Mehrzahl von durch frühere Benutzer abgearbeiteten Prozesse (20) für die Herstellung von Katalysatoren und deren gemessenen Eigenschaften (25, 26, 27) eine Korrelation von wenigstens einem Prozessparameter (21, 22, 23) eines Prozesses (20) für die Herstellung eines Katalysators und wenigstens einer Eigenschaft (25, 26, 27) des Katalysators herzustellen

15. Vorrichtung gemäß Anspruch 14, wobei auf der Grundlage der Korrelation und der durch den Benutzer vorgenommenen Schritte (11', 12', 13') bei einer Abarbeitung des ausgewählten Prozesses (10) eine Prognose für wenigstens eine Eigenschaft (15, 16, 17) des herzustellenden Katalysators erstellt wird.

## Claims

1. A computer-implemented method of monitoring and evaluating a production of a functional material, wherein the functional material is a catalyst, and wherein the method comprises:
providing (S10) a multitude of defined processes (10) to a user (100), each with defined process parameters (11, 12, 13) of a process (10) for the production of a catalyst and properties thereof (15, 16, 17),
recording (S20) steps (11', 12', 13') taken by the user in implementing a process (10) selected from the multitude of processes,
comparing (S50) the steps (11', 12', 13') taken by the user with the process parameters (11, 12, 13) of the selected process (10) for the production of a catalyst,
assessing (S60) the steps (11', 12', 13') taken by the user based on a data basis having a multitude of processes (20) implemented by earlier users with process parameters (21, 22, 23) for the production of catalysts and the analyzed properties thereof (25, 26, 27),
reporting (S70) to the user the extent to which predetermined properties (15, 16, 17) of the catalyst produced by the implemented process (10) are attained in the event of variances of the steps (11', 12', 13') taken in an implementation of the process selected from the defined process parameters (11, 12, 13) of the selected process (10).

2. The method according to claim 1, wherein the method further comprises assistance (S30) for a user (100) in a projection calculation, wherein the assistance especially comprises calculating (S31) of relationships and/or recalculating (S32) of parameters, wherein, in particular, at least some of the processes (10) are scalable and a user (100) is given the choice of dimensions of these processes (10).

3. The method according to either of claims 1 and 2, wherein the method further comprises monitoring (S40) a user (100) in implementing the steps taken in a process (10) selected from the multitude of processes.

4. The method according to claim 3, wherein the monitoring (S40) comprises comparing (S42) at least one step (11', 12', 13') intended by the user with an appropriate process parameter (11, 12, 13) of a selected process (10) for the production of a catalyst, and a warning (S43) in the event of an impermissible variance.

5. The method according to either of claims 3 and 4, wherein the monitoring (S40) comprises reading (S45) of an identifier of a reservoir vessel of a material provided by a user and a comparison (S46) with a material provided for the corresponding defined process, and a warning (S47) in the event of an impermissible variance.

6. The method according to any of the preceding claims, wherein a process parameter or a step (11, 11', 12, 12', 13, 13') taken by the user comprises at least one material parameter and one processing parameter, and/or an origin parameter of a material.

7. The method according to any of the preceding claims, wherein the assessing (S60) of the steps taken by the user (100) comprises employing (S65) of artificial intelligence which, on the basis of the multitude of processes (20) implemented by earlier users stored in the data basis for the production of catalysts and the measured properties thereof (25, 26, 27), establishes a correlation of at least one process parameter (11, 12, 13) of a process (10) for the production of a catalyst and at least one property of the catalyst (25, 26, 27).

8. The method according to claim 7, wherein, on the basis of the correlation and the steps (11', 12', 13') taken by the user in an implementation of the selected process (10), a prediction is created for at least one property (15, 16, 17) of the functional material to be produced.

9. The method according to any of the preceding claims, wherein the assessing (S60) of the steps taken by the user (100) comprises an intermediate assessment (S61, S62) on the basis of an intermediate product based on a data basis comprising a multitude of processes implemented by earlier users for the production of precursor materials and the measured properties thereof.

10. A computer-implemented method of establishing a data basis for employment of a method according to any of claims 1 to 10, wherein the method comprises:
recording (S90) steps (21, 22, 23) taken by users in an implementation of a process (20) for production of a catalyst,
analyzing (S92) properties (25, 26, 27) of the catalyst produced by the process (20) for producing a catalyst,
assigning (S96) the properties (25, 26, 27) of the catalyst produced by the process (20) for producing a catalyst to the corresponding steps (21, 22, 23) in an implementation of the process (20) for producing a catalyst,
storing (S98) the result of the assignment in the data basis.

11. The method according to claim 10, wherein the method of establishing a data basis comprises employing (S94) of artificial intelligence which, on the basis of a multitude of recordings (S90) of steps (21, 22, 23) taken by users (100) in an implementation of a process (20) for producing a catalyst and the analyzed properties thereof (25, 26, 27), establishes a correlation of at least one process parameter (21, 22, 23) of a process (20) for the production of a catalyst and at least one property (25, 26, 27) of the analyzed catalyst.

12. An apparatus for monitoring and evaluating a production of a functional material, wherein the functional material is a catalyst, and wherein the apparatus comprises:
a user interface (110),
a processing unit (120),
a catalog (130) having a multitude of defined processes (10), each with defined process parameters (11, 12, 13) of a process for the production of a catalyst, and
a data basis (140) having a multitude of processes (20) implemented by earlier users for the production of catalysts and the analyzed properties thereof (25, 26, 27),
wherein the user interface (110) is set up to provide the user (100) with the catalog (130) for selection, and to enable the user to select a process (10) for the production of a catalyst,
wherein the user interface (110) is set up to record steps (11', 12', 13') taken by the user in implementing a process (10) selected from the multitude of processes,
wherein the processing unit (120) is set up to compare the steps (11', 12', 13') taken by the user with the process parameters (11, 12, 13) of a selected process (10) for the production of a catalyst and to assess the steps (11', 12', 13') taken by the user based on the data basis (140) with regard to properties expected to be achieved in the catalyst,
wherein the user interface (110) is set up to report to the user the extent to which predetermined properties (15, 16, 17) of the catalyst produced by the implemented process (10) are attained in the event of variances of the steps (11', 12', 13') taken in an implementation of the process selected from the defined process parameters (11, 12, 13) of the selected process (10).

13. The apparatus according to claim 12, wherein the apparatus includes a monitoring device (150) set up to compare at least one step (11', 12', 13') intended by the user (100) with an appropriate process parameter (11, 12, 13) of a selected process (10) for the production of a catalyst, and to give a warning to the user in the event of an impermissible variance.

14. The apparatus according to either of claims 12 and 13, wherein the processing unit (120) comprises artificial intelligence (125) set up to establish, on the basis of the multitude of processes (20) implemented by earlier users stored in the data basis (140) for the production of catalysts and the measured properties thereof (25, 26, 27), a correlation of at least one process parameter (21, 22, 23) of a process (20) for the production of a catalyst and at least one property (25, 26, 27) of the catalyst.

15. The apparatus according to claim 14, wherein, on the basis of the correlation and the steps (11', 12', 13') taken by the user in an implementation of the selected process (10), a prediction is created for at least one property (15, 16, 17) of the catalyst to be produced.

## Revendications

1. Procédé mis en œuvre par ordinateur pour surveiller et évaluer la production d'un matériau fonctionnel, le matériau fonctionnel étant un catalyseur, et le procédé comprenant :
la fourniture (S10) d'une pluralité de processus (10) prédéfinis à un utilisateur (100) avec des paramètres de processus (11, 12, 13) prédéfinis respectifs d'un processus (10) de production d'un catalyseur et de ses propriétés (15, 16, 17),
la détection (S20) d'étapes (11', 12', 13') effectuées par l'utilisateur lors de l'exécution d'un processus (10) sélectionné parmi la pluralité de processus,
la comparaison (S50) des étapes (11', 12', 13') effectuées par l'utilisateur avec les paramètres de processus (11, 12, 13) du processus (10) sélectionné de production d'un catalyseur,
l'évaluation (S60) des étapes (11', 12', 13') effectuées par l'utilisateur sur la base d'une base de données qui comprend une pluralité de processus (20) exécutés par des utilisateurs précédents avec des paramètres de processus (21, 22, 23) de production de catalyseurs et leurs propriétés analysées (25, 26, 27),
le signalement en retour (S70) à l'utilisateur de la mesure dans laquelle des propriétés prédéterminées (15, 16, 17) du catalyseur produit par le processus (10) exécuté sont atteintes en cas d'écarts des étapes (11', 12', 13') effectuées lors d'une exécution du processus sélectionné par rapport aux paramètres de processus (11, 12, 13) prédéfinis du processus (10) sélectionné.

2. Procédé selon la revendication 1, le procédé comprenant en outre une assistance (S30) à un utilisateur (100) lors d'un calcul de la charge, l'assistance comprenant en particulier un calcul (S31) de rapports et/ou une conversion (S32) de paramètres, au moins une partie des processus (10) étant en particulier évolutive et offrant à un utilisateur (100) le choix de dimensionner ces processus (10).

3. Procédé selon l'une des revendications 1 et 2, le procédé comprenant en outre une surveillance (S40) d'un utilisateur (100) lors de l'exécution des étapes effectuées d'un processus (10) sélectionné parmi la pluralité de processus.

4. Procédé selon la revendication 3, dans lequel la surveillance (S40) comprend une comparaison (S42) d'au moins une étape (11', 12', 13') envisagée par l'utilisateur avec un paramètre de processus (11, 12, 13) correspondant d'un processus (10) sélectionné de production d'un catalyseur, et un avertissement (S43) en cas d'écart inacceptable.

5. Procédé selon l'une des revendications 3 et 4, dans lequel la surveillance (S40) comprend une lecture (S45) d'un identifiant d'un réservoir d'un matériau prévu par un utilisateur et une comparaison (S46) avec un matériau prévu pour le processus prédéfini correspondant, ainsi qu'un avertissement (S47) en cas d'écart inacceptable.

6. Procédé selon l'une des revendications précédentes, dans lequel un paramètre de processus ou une étape (11, 11', 12, 12', 13, 13') effectuée par l'utilisateur comprend au moins un paramètre de matériau et un paramètre de traitement, et/ou comprend un paramètre d'origine d'un matériau.

7. Procédé selon l'une des revendications précédentes, dans lequel l'évaluation (S60) des étapes effectuées par l'utilisateur (100) comprend une application (S65) d'une intelligence artificielle qui, sur la base de la pluralité de processus (20) de production de catalyseurs exécutés par des utilisateurs précédents et stockés dans la base de données et de leurs propriétés mesurées (25, 26, 27), établit une corrélation entre au moins un paramètre de processus (11, 12, 13) d'un processus (10) de production d'un catalyseur et au moins une propriété du catalyseur (25, 26, 27).

8. Procédé selon la revendication 7, dans lequel, sur la base de la corrélation et des étapes (11', 12', 13') effectuées par l'utilisateur lors d'une exécution du processus (10) sélectionné, une prévision est établie pour au moins une propriété (15, 16, 17) du matériau fonctionnel devant être produit.

9. Procédé selon l'une des revendications précédentes, dans lequel l'évaluation (S60) des étapes effectuées par l'utilisateur (100) comprend une évaluation intermédiaire (S61, S62) basée sur un produit intermédiaire sur la base d'une base de données qui comprend une pluralité de processus de production de matériaux précurseurs exécutés par des utilisateurs précédents et leurs propriétés mesurées.

10. Procédé mis en œuvre par ordinateur pour construire une base de données pour l'application d'un procédé selon l'une des revendications 1 à 10, ledit procédé comprenant :
la détection (S90) d'étapes (21, 22, 23) effectuées par des utilisateurs lors d'une exécution d'un processus (20) de production d'un catalyseur,
l'analyse (S92) de propriétés (25, 26, 27) du catalyseur produit par le processus (20) de production d'un catalyseur,
l'attribution (S96) des propriétés (25, 26, 27) du catalyseur produit par le processus (20) de production d'un catalyseur aux étapes (21, 22, 23) correspondantes lors d'une exécution du processus (20) de production d'un catalyseur,
le stockage (S98) du résultat de l'attribution dans la base de données.

11. Procédé selon la revendication 10, dans lequel le procédé de construction d'une base de données comprend une application (S94) d'une intelligence artificielle qui, sur la base d'une pluralité de détections (S90) d'étapes (21, 22, 23) effectuées par des utilisateurs (100) lors de l'exécution d'un processus (20) de production d'un catalyseur et de ses propriétés (25, 26, 27) analysées, établit une corrélation entre au moins un paramètre de processus (21, 22, 23) d'un processus (20) de production d'un catalyseur et au moins une propriété (25, 26, 27) du catalyseur analysé.

12. Appareil pour surveiller et évaluer la production d'un matériau fonctionnel, le matériau fonctionnel étant un catalyseur et l'appareil comprenant :
une interface utilisateur (110),
une unité de traitement (120),
un catalogue (130) comprenant une pluralité de processus (10) prédéfinis avec des paramètres de processus (11, 12, 13) prédéfinis d'un processus de production d'un catalyseur, et
une base de données (140) comprenant une pluralité de processus (20) de production de catalyseurs exécutés par des utilisateurs précédents et leurs propriétés (25, 26, 27) analysées,
l'interface utilisateur (110) étant conçue pour fournir à l'utilisateur (100) le catalogue (130) destiné à effectuer la sélection et pour permettre à l'utilisateur de sélectionner un processus (10) de production d'un catalyseur,
l'interface utilisateur (110) étant conçue pour détecter des étapes (11', 12', 13') effectuées par l'utilisateur lors de l'exécution d'un processus (10) sélectionné parmi la pluralité de processus,
l'unité de traitement (120) étant conçue pour comparer les étapes (11', 12', 13') effectuées par l'utilisateur avec les paramètres de processus (11, 12, 13) d'un processus (10) sélectionné de production d'un catalyseur et pour évaluer les étapes (11', 12', 13') effectuées par l'utilisateur sur la base de la base de données (140) en ce qui concerne les propriétés du catalyseur qui seront probablement obtenues,
l'interface utilisateur (110) étant conçue pour signaler en retour à l'utilisateur la mesure dans laquelle des propriétés (15, 16, 17) prédéfinies du catalyseur produit par le processus (10) exécuté sont atteintes en cas d'écarts des étapes (11', 12', 13') effectuées lors d'une exécution du processus sélectionné par rapport aux paramètres de processus (11, 12, 13) prédéfinis du processus (10) sélectionné.

13. Appareil selon la revendication 12, dans lequel l'appareil comprend un dispositif de surveillance (150) conçu pour comparer au moins une étape (11', 12', 13') envisagée par l'utilisateur (100) avec un paramètre de processus correspondant (11, 12, 13) d'un processus (10) sélectionné de production d'un catalyseur et pour délivrer à l'utilisateur un avertissement en cas d'écart inacceptable.

14. Appareil selon l'une des revendications 12 à 13, dans lequel l'unité de traitement (120) comprend une intelligence artificielle (125) conçue, sur la base de la pluralité de processus (20) de production de catalyseurs exécutés par des utilisateurs précédents et stockés dans la base de données (140) et de leurs propriétés (25, 26, 27) mesurées, pour établir une corrélation entre au moins un paramètre de processus (21, 22, 23) d'un processus (20) de production d'un catalyseur et au moins une propriété (25, 26, 27) du catalyseur.

15. Appareil selon la revendication 14, dans lequel, sur la base de la corrélation et des étapes (11', 12', 13') effectuées par l'utilisateur lors d'une exécution du processus (10) sélectionné, une prévision est établie pour au moins une propriété (15, 16, 17) du catalyseur devant être produit.
